# EUROPÄISCHE PATENTSCHRIFT

(11) **EP 0 492 536 B1**
(45) Veröffentlichungstag und Bekanntmachung des Hinweises auf die Patenterteilung: **14.05.2003**
(21) Anmeldenummer: 91122006.9
(22) Anmeldetag: 20.12.1991
(51) Int. Cl.: C12N 15/82, C12N 15/29, A01H 5/00, A61K 38/16

(54) **Verfahren zur Erzeugung von pathogen-resistenten Pflanzen**
Method for the production of pathogen resistant plants
Méthode de production de plantes résistantes aux pathogènes

(30) Priorität: 20.12.1990 DE 4040954
(43) Veröffentlichungstag der Anmeldung: 01.07.1992
(60) Teilanmeldung: 98117774.4
(73) Patentinhaber: Max-Planck-Gesellschaft zur Förderung der Wissenschaften e.V., 80539 München (DE)
(72) Erfinder: Schell, Jeff, Prof. Dr., W-5000 Köln 30 (DE); Logemann, Jürgen, Dr., W-5000 Köln 30 (DE); Jach, Guido, Dipl.-Biologe, W-5000 Köln 1 (DE); Mundy, John, Dr., DK-1760 Copenhagen V. (DK)
(74) Vertreter: von Hellfeld, Axel, Dr. Dipl.-Phys.

(56) Entgegenhaltungen:
- EP-A- 0 298 918
- EP-A- 0 375 091
- WO-A-90/12097
- CHEMICAL ABSTRACTS, vol. 113, 1990, Columbus, Ohio, US; abstract no. 127723, JUN, WANG: 'Preparation of transgenic plants for control of virosis' Seite 205 ;
- CARLSBERG RES. COMMUN. Bd. 51, Nr. 3, 1986, Seiten 129 - 141; ASANO, K., ET AL.: 'The complete primary structure of the protein synthesis inhibitor II from barley seeds'
- BIOCONJUGATE CHEMISTRY Bd. 1, Nr. 5, September 1990, Seiten 331 - 336; EBERT, R.F., ET AL.: 'Immunotoxin construction with a ribosome-inactivating protein from barley'
- JOURNAL OF BIOLOGICAL CHEMISTRY. Bd. 266, Nr. 3, 25. Januar 1991, BALTIMORE US Seiten 1564 - 1573; LEAH, R., ET AL.: 'Biochemical and molecular characterization of three barley seed proteins with antifungal properties'

## Beschreibung

Die Erfindung betrifft ein Verfahren zur Erzeugung von pathogenresistenten Pflanzen, nach dem Verfahren erzeugte Pflanzen und Pflanzenteile, neue DNA-Übertragungsvektoren und DNA-Expressionsvektoren sowie schließlich die Verwendung eines Proteinsynthese-Inhibitorproteins zur Herstellung pharmazeutischer Präparate.

### Stand der Technik

Es ist bekannt, z. B. aus Ann.Rev.Plant Physiol. 1979, 30: 105-130 sowie Ann.Rev.Plant Physiol. 1984, 35:34-275, daß Pflanzen die verschiedensten Mechanismen benutzen, um sich gegen Infektionen durch Pathogene zu schützen. Zu diesen Mechanismen gehören z. B. Veränderungen in der Zellwandstruktur, die Synthese von toxisch wirkenden Phytoalexinen, die Akkumulation von sogenannten PR-Proteinen (Pathogenesis-related Proteins), Protease-Inhibitoren sowie Enzyme mit hydrolytischen Funktionen.

Es ist ferner bekannt, z. B. aus Biochem. J. 1983, 216:617-625, daß verschiedene Pflanzen Proteine zu erzeugen vermögen, die die Fähigkeit besitzen, die Ribosomen von Eukaryonten zu inhibieren. Charakteristisch für solche die Proteinsynthese inhibierenden Proteine ist die Eigenschaft, daß sie pflanzeneigene Ribosomen nicht beeinflussen, während sie pflanzenfremde Ribosomen inaktivieren. Derartige Proteine sind insbesondere unter der Bezeichnung "RIP"-Proteine (Ribosomen inhibierende Proteine) bekannt geworden. Von den meisten dieser Proteine sind lediglich ihr Molekulargewicht und ihre Wirkungsweise bekannt.

Zu den Pflanzen, in denen RIP-Proteine gefunden wurden, gehört u. a. auch die Gerste. So werden in Carlsberg Res. Commun. Vol. 51,1986, S. 129-141 das gereinigte Protein, das Molekulargewicht desselben wie auch die Aminosäuresequenz des Proteinsynthese-Inhibitors II beschrieben.

Es ist ferner bekannt, z. B. aus Biochimica et Biophysica Acta 880, 1986, S. 161-170, daß RIP-Proteine "in vitro" Pathogene zu inhibieren vermögen.

### Erfindung

Bei der Untersuchung von insbesondere Gerstepflanzen wurden die Gene identifiziert, die für Protein-Synthese-Inhibitoren (PSI) codieren. Es wurde gefunden, daß diese PSI-Gene für PSI-Proteine codieren, die die Proteinsynthese von Pflanzenpathogenen wirksam zu blockieren vermögen.

Es wurde ferner gefunden, daß sich z. B. aus Gerstepflanzen isolierte PSI-Gene mit den verschiedensten aktiven Promotoren, beispielsweise dem wun1-Promotor, der näher beschrieben wird in "The Plant Cell 1", 1989, S.151-158 fusionieren lassen und daß solche Promotor-Gen-Fusionen in das Erbgut von Pflanzen eingebaut und transgene Pflanzen erzeugt werden können, die neu erworbene Pathogenresistenz zeigen.

Weiterhin wurde gefunden, daß sich das PSI-Protein auch zur Herstellung pharmazeutischer Präparate verwenden läßt, die zur Behandlung von Menschen und Tieren eingesetzt werden können, die von pilzlichen, bakteriellen, viralen oder anderen Pathogenen befallen sind.

Durch die Einführung des PSI-Gens in bakterielle Überproduzenten läßt sich das PSI-Protein in großen Mengen herstellen. Gereinigtes PSI-Protein läßt sich in Form von Infusionslösungen in die Blutbahn des Menschen oder des Tieres einführen. Das PSI-Protein inhibiert das Pathogen (wie z. B. Aidsviren), ohne den Organismus zu schädigen. Die Pathogenspezifität des PSI-Proteins läßt sich gegebenennfalls weiter dadurch erhöhen, daß das PSI-Protein mit pathogenspezifischen Antikörpern gekoppelt wird.

Auch die Behandlung von entarteten Zellen (Krebs) bei Menschen oder Tieren ist durch die Verwendung von PSI-Protein möglich. So läßt sich gereinigtes PSI-Protein oder PSI-Protein, das mit Antikörpern gekoppelt wurde, die spezifisch entartete Zellen erkennen, in die Blutbahn einführen, um entartete Zellen zu zerstören. Andere Darreichungsformen, z. B. in Kapseln sind möglich.

Geeignete Infusionslösungen lassen sich dabei nach Methoden herstellen, wie sie für die Herstellung wäßriger Infusionslösungen üblich und bekannt sind.

Gegenstand der Erfindung sind demzufolge ein Verfahren zur Erzeugung von pathogen-resistenten Pflanzen, wie es in den Ansprüchen gekennzeichnet ist, neue DNA-Übertragungsvektoren und DNA-Expressionsvektoren sowie Pflanzen und Pflanzenteile, die nach dem erfindungsgemäßen Verfahren feststellbar sind.

Gegenstand der Erfindung ist ferner die Verwendung des durch Einführung des Proteinsynthese-Inhibitorgens in bakterielle Überproduzenten gewonnenen Proteinsynthese-Inhibitorproteins zur Herstellung pharmazeutischer Präparate für die Erzeugung pathogener Resistenzen, zur Bekämpfung von pathogenem Befall und/oder entarteten Zellen.

Typische Pflanzenpathogene, deren Proteinsynthese durch Einbau eines PSI-Gens gehemmt werden kann, sind beispielsweise die Pilze Trichoderma reesei und Fusarium sporotrichioides. (Verwiesen wird auf die Übersicht in M. Klinkowski, E. Mühle, E. Reinmuth und H. Bochow: "Phytopathologie und Pflanzenschutz I + II", Akademie-Verlag, Berlin, 1974.)

Fusarienpilze befallen bekanntlich hauptsächlich Getreidearten und Mais-Pflanzen, während Pilze der Gattung Trichoderma vorwiegend auf Maiskörnern zu finden sind.

Überraschenderweise hat sich gezeigt, daß die pathogen-hemmenden Eigenschaften von, z. B. aus Gerstepflanzen, isolierten PSI-Genen auch auf artfremde Pflanzen übertragbar sind. So wurde beispielsweise gefunden, daß ein aus Gerste isoliertes PSI-Gen unter der Kontrolle eines aktiven Promotors, z. B. des wunl-Promotors, in das Erbgut von Tabakpflanzen eingebaut werden kann. Tabakpflanzen, die daraufhin das PSI-Protein produzieren, zeigen neu erworbene Resistenzeigenschaften gegen z. B. das Pflanzenpathogen Rhizoctonia solani. Rhizoctonia bewirkt die sogenannte Wurzeltöterkrankheit (Befall von Stengel und Wurzel) bei diversen Pflanzen, inklusive Kartoffel- und Tabakpflanzen. Somit sind neuerworbene Resistenzeigenschaften in Pflanzen direkt mit der Expression von PSI-Genen korreliert.

Außer aus Gerste lassen sich Proteinsynthese-Inhibitor-Gene beispielseise aus allen monocotylen und dicotylen Pflanzen isolieren und mit den verschiedensten aktiven Promotoren fusionieren, wie beispielsweise pathogeninduzierbaren Promotoren, konstitutiven Promotoren, entwicklungsspezifischen Promotoren, organspezifischen Promotoren und induzierbaren Promotoren.

Als Pflanzen, in deren Erbgut die neuen Proteinsynthese-Inhibitorgene unter der Kontrolle eines aktiven Promotors eingebaut werden können, seien beispielsweise genannt:
- alle monocotylen Nutzpflanzen, wie z. B. Getreidepflanzen
- alle dicotylen Nutzpflanzen, wie z. B. Solanaceen und Cucurbitaceen.

Insbesondere eignet sich das erfindungsgemäße Verfahren somit zur Erzeugung von pathogenresistenten Pflanzen. Durch die Expression des Proteinsynthese-Inhibitorgens läßt sich jedoch auch beim Menschen und bei Tieren eine Resistenz gegenüber Insekten, Pilzen, Bakterien, Viren und Viroiden erreichen.

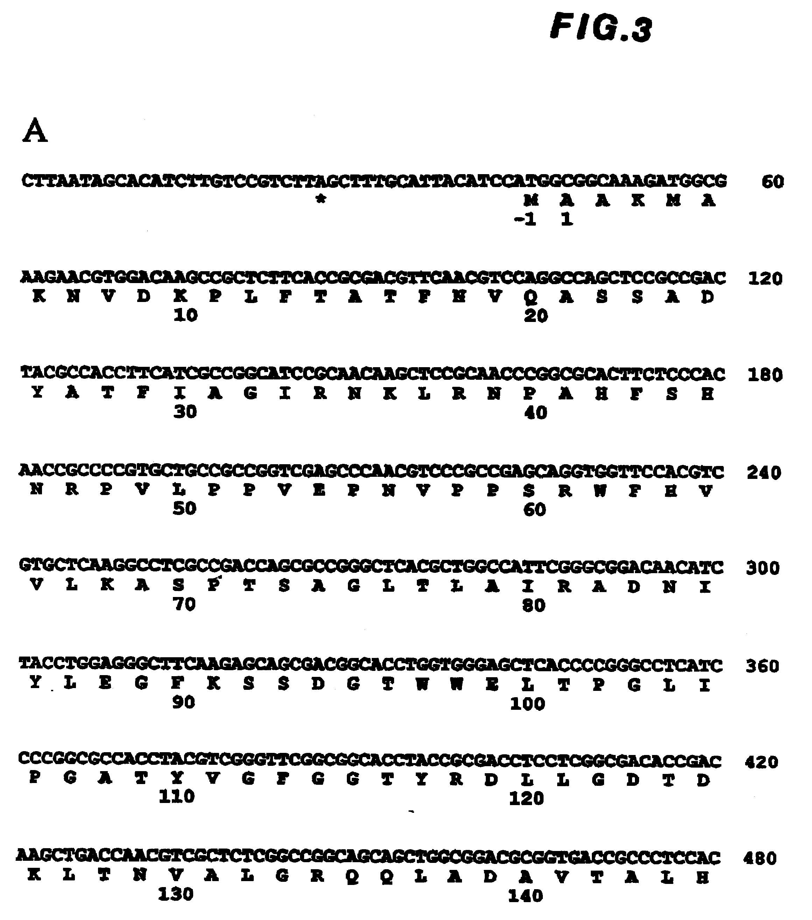

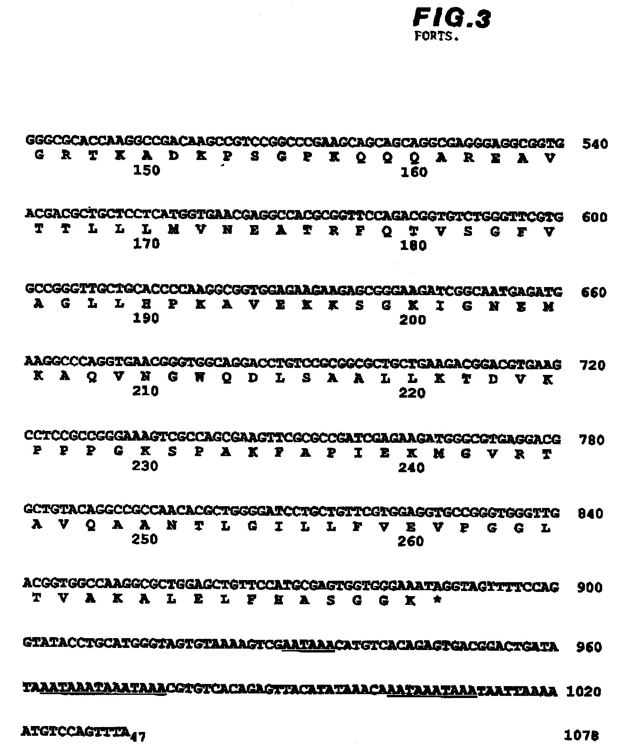

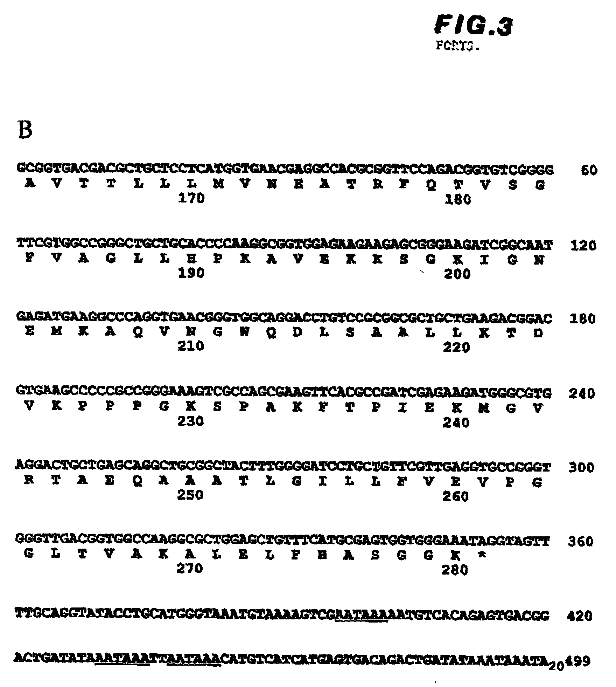

Zur Durchführung des erfindungsgemäßen Verfahrens eignet sich gemäß einer vorteilhaften Ausgestaltung ein Proteinsynthese-Inhibitorgen, das die in Fig. 3A dargestellte DNA-Sequenz aufweist. Für den Fachmann ergibt sich jedoch, daß außer dieser DNA-Sequenz auch ähnliche DNA-Sequenzen zur Lösung der gestellten Aufgabe verwendet werden können, z. B. eine DNA-Sequenz gemäß Fig. 3B, die im 5'-Bereich durch einen entsprechenden cDNA-Klon komplettiert worden ist.

Die Erfindung wird im folgenden beispielsweise anhand der Isolierung eines PSI-Gens aus Gerste, Fusion dieses Gens mit einem aktiven Promotor und Transfer des Fusionsproduktes in das Erbgut von Tabakpflanzen näher erläutert.

Die Figuren dienen der näheren Erläuterung der Erfindung. Im einzelnen sind dargestellt in:

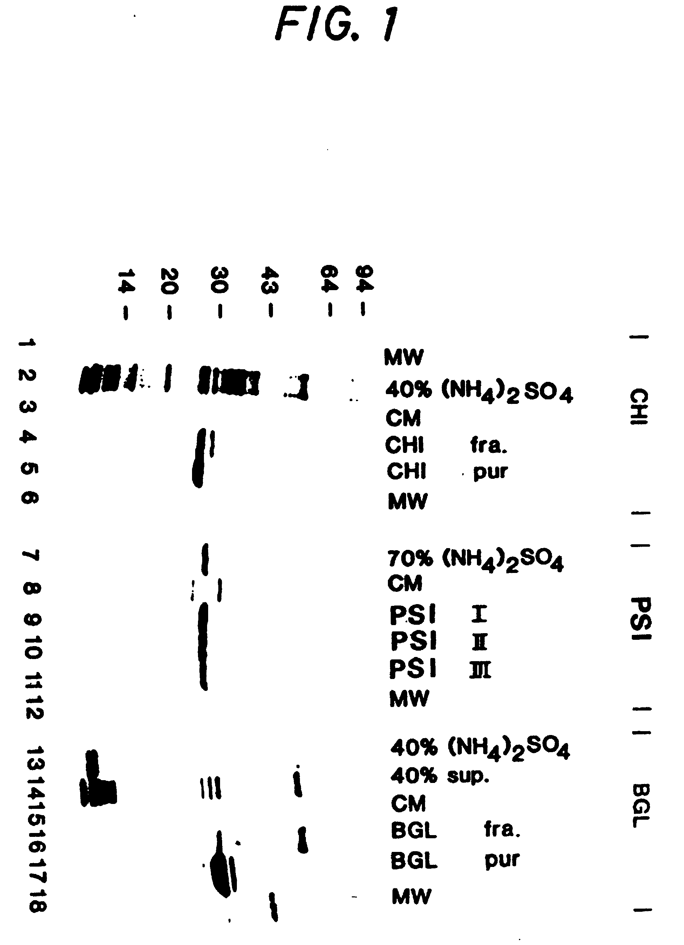

Fig. 1 Reinigung von CHI-, BGL- und PSI-Proteinen aus Gerste
   Gezeigt wird die SDS-Gel-elektrophoretische Auftrennung von Proteinfraktionen, die bei der Reinigung von CHI-Protein (Reihe 2-5) PSI-Protein (Reihe 7-11) und BGL-Protein (Reihe 13-17) aus Gerstesamen entstanden sind. Mit Hilfe von spezifischen Antikörpern können die entsprechenden Proteine nachgewiesen werden. Dabei bedeuten:
   % (NH₄)₂SO₄: Proteine, die durch das Salz gefällt worden sind.
   % sup.: Proteine, die durch das Salz nicht gefällt worden sind.
   CM: Proteine, die durch die CM-Säule nicht gebunden worden sind.
   fra.: Proteinfraktion aus CM-Säule.
   pur.: hochreines Protein.
   MW: Protein-Größenstandard in kDalton.

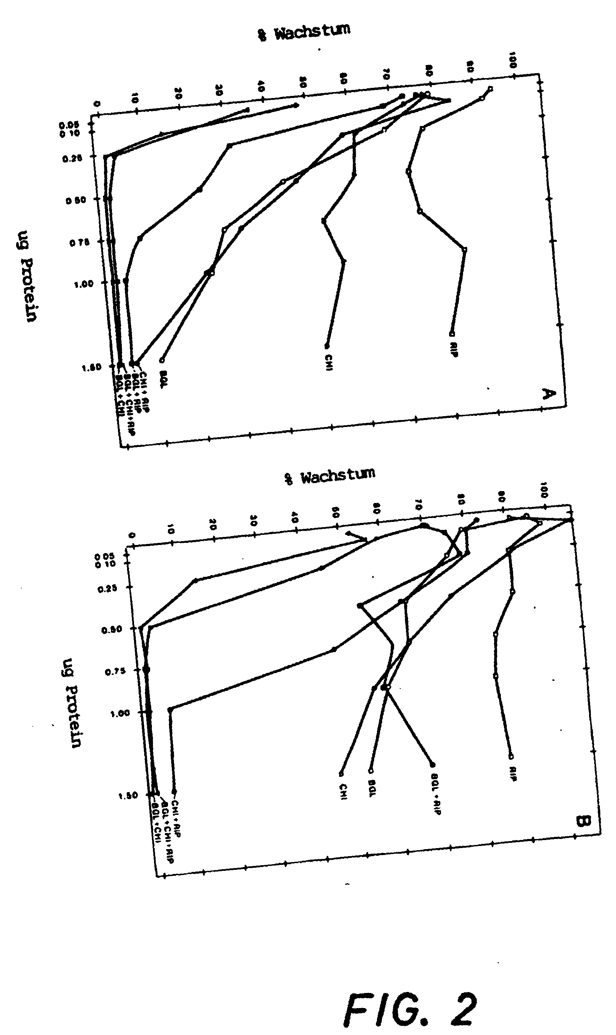

Fig. 2 Pilzwachstumstest mit gereinigtem Protein
   Sporen von Trichoderma reesei (A) und Fusarium sporotrichioides (B) wurden in einem Gesamtvolumen von 135 µl Medium/Loch einer Mikrotiterplatte angezogen und mit 0,05 - 1,5 µg des jeweils angegebenen Proteins versetzt. Jede Punktmarkierung ist das Resultat aus 5 unabhängigen Messungen mit relativen Standardabweichungen von 3,6 % bei (A) und 7,3 % bei (B). 100 % Pilzwachstum führt zu einer O.D.₅₄₀ von 0,40 (A) und 0,41 (B).
Fig. 3 Nukleotidsequenz der isolierten PSI-cDNA-Klone
   A: Der cDNA-Klon ist 1078 Nukleotide groß. Er enthält einen 42 bp großen 5'-untranslatierten Bereich, ein offenes Leseraster von 843 bp (das Stoppcodon ist mit * markiert), und ein 193 bp großes 3'-untranslatiertes Ende. Mögliche Polyadenylierungssignale sind unterstrichen. Die Aminosäuresequenz, die sich aus dem offenen Leseraster ergibt, ist unterhalb der entsprechenden Sequenz angegeben.
   B: Nukleotidsequenz eines unvollständigen PSI-cDNA-Klons. Mögliche Polyadenylierungssignale sind unterstrichen.

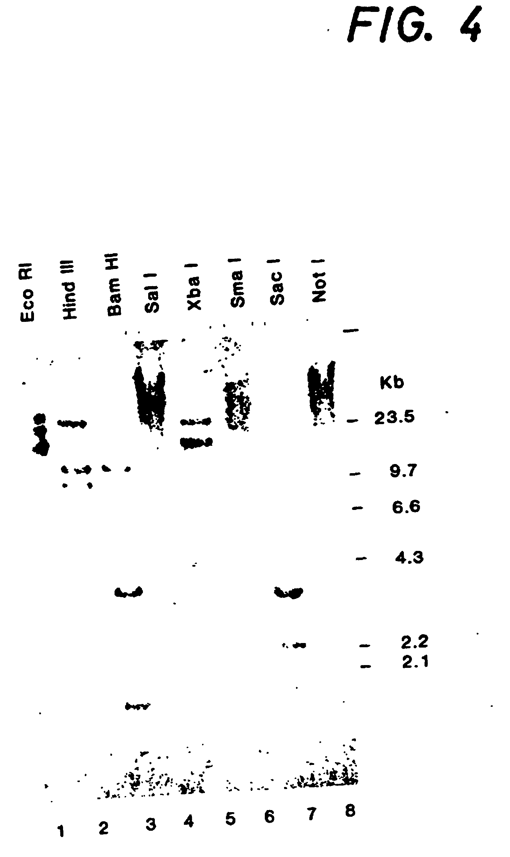

Fig. 4 Organisation von PSI-Genen im Gerste-Genom
   DNA aus Gerste-Embryos wurde mit verschiedenen Restriktionsenzymen geschnitten, gelektrophoretisch aufgetrennt, auf Nylonmembranen transferiert und gegen radioaktiv markierte PSI-cDNA hybridisiert. Aufgrund der Anzahl der hybridisierenden Banden können Rückschlüsse auf die PSI-Kopienanzahl im Genom gemacht werden. Der Größenstandard ist in Kilobasenpaaren (Kb) angegeben.

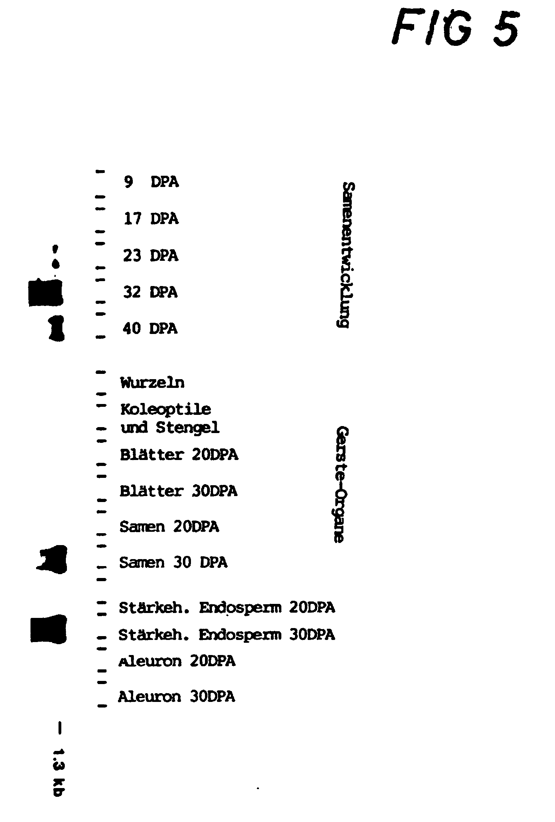

Fig. 5 Entwicklungs- und organspezifische Expression von PSI-RNA in Gerste
   Aus verschiedenen Organen von Gerstepflanzen sowie zu unterschiedlichen Entwicklungsstadien von Gerste-Samen wurde RNA isoliert. Die RNA wurde nach gelelektrophoretischer Auftragung über die "Northern Blot Methode" gegen radioaktiv markierte PSI-cDNA hybridisiert. PSI-RNA ist als 1,3 Kb große RNA spezifisch im stärkehaltigen Endosperm während der späten Samenentwicklung nachweisbar. DPA steht dabei für Tage nach der Anthese.

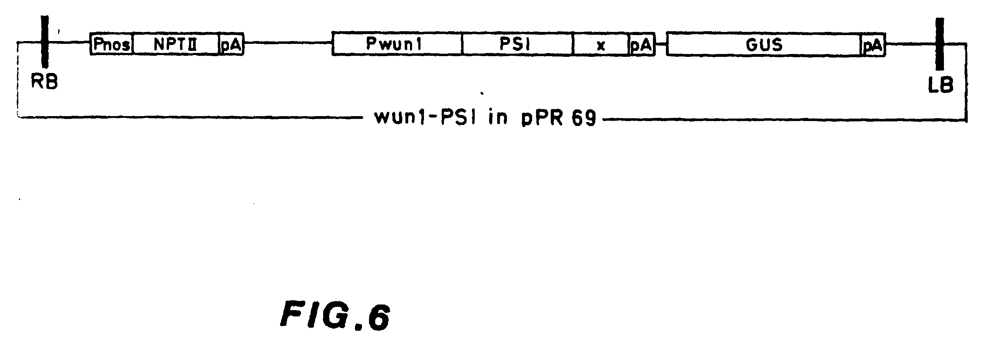

Fig. 6 Konstruktionskarte des chimären Gens wunl-PSI in pPR69
   Der wun1-Promotor ("Pwun1"; ca. 1200 bp groß) ist transkriptionell mit dem PSI-Gen ("PSI"; ca. 1070 bp groß) fusioniert worden. Aus RNA-Stabilitätsgründen ist 3' vom PSI-Gen ein Rest des CAT-Gens ("x"; ca. 500 bp groß) sowie ein Polyadenylierungssignal ("pA"; ca. 200 bp groß) fusioniert worden. Dieses Konstrukt wurde in den binären Vektor pPR69 kloniert.

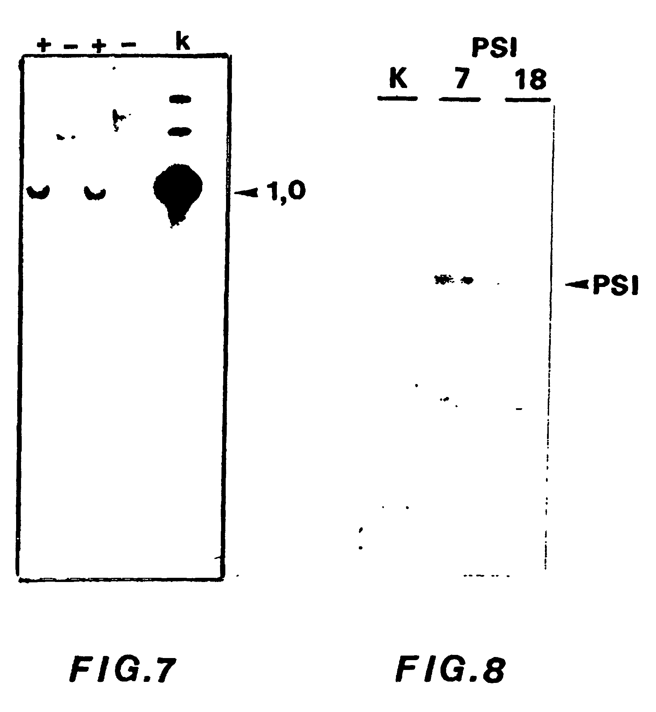

Fig. 7 Southern-Blot-Analyse wunl-PSI-transgener Tabakpflanzen
   Um die korrekte Integration von wun1-PSI-DNA in das Tabakgenom analysieren zu können, wurde DNA von wun1-PSI-transgenen Tabakpflanzen isoliert und mit EcoRI geschnitten. Nach der gelelektrophoretischen Auftrennung der DNA sowie dem Transfer der DNA auf Nylonmembranen wurde gegen radioaktive PSI-cDNA hybridisiert. Mit einem (+) bezifferte Pflanzen zeigen eine korrekte Integration der wunl-PSI-DNA. Die Größe der hybridisierenden Bande beträgt 1,07 Kb und ist identisch mit der Größe der zur Kontrolle aufgetragenen Plasmid-DNA von wunl-PSI in pPR69 (K). Mit (-) bezifferte Pflanzen wurden aufgrund des Vorhandenseins zusätzlicher oder inkorrekter großer DNA-Banden verworfen.
Fig. 8 Northern-Blot-Analyse wunl-PSI-transgener Tabakpflanzen
   100 µg Blatt-RNA aus Rhizoctonia solani-infizierten Tabakpflanzen wurde gelelektrophoretisch aufgetrennt, auf Nylonmembranen transferiert und gegen radioaktive PSI-cDNA hybridisiert. Auf der Autoradiografie ist bei wun1-PSI transgenen Tabakpflanzen ("PSI-7; PSI-18) eine PSI-RNA-Bande erkennbar. In untransformierten Tabakpflanzen (K) ist keine PSI-RNA nachweisbar.

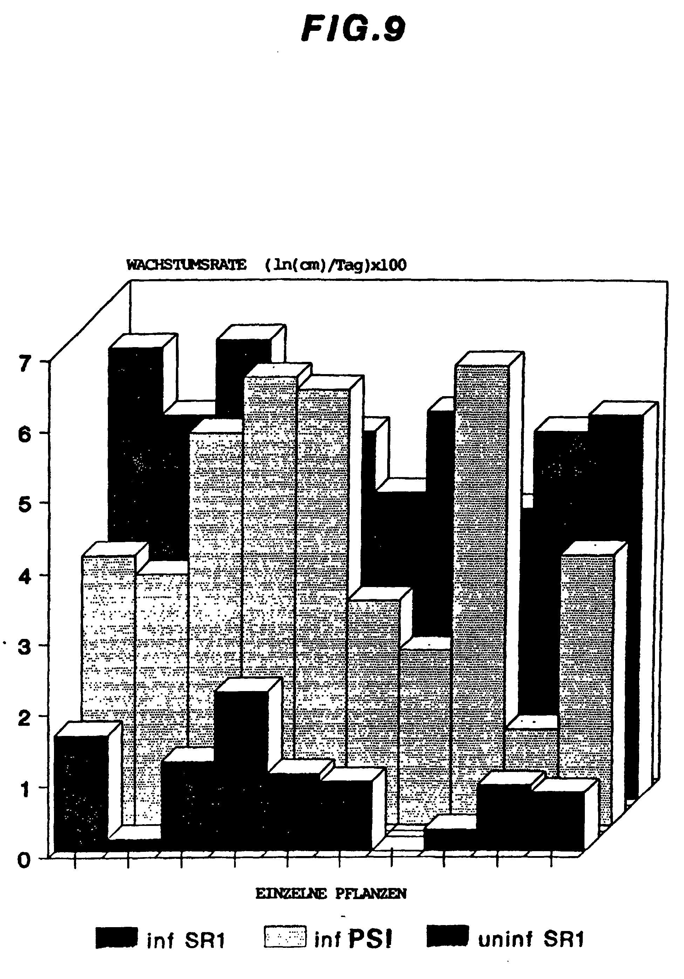

Fig. 9 Wachstumsrate einzelner mit Rhizoctonia solaniinfizierter Tabakpflanzen
   Jeder dargestellte Balken repräsentiert das Wachstum einer Tabakpflanze über einen Zeitraum von ca. 10 Tagen. Aus Steigung der Wachstumskurve wurde der ln×100 errechnet, so daß der entstehende Wert die Wachstumsrate repräsentiert.
   "inf. SRI": untransformierter Tabak, der mit Rhizoctonia solani infiziert wurde. "inf. RIP: wunl-PSI transgener Tabak, der mit Rhizoctonia solani infiziert wurde. "uninf. SRI: untransformierter Tabak, der nicht infiziert wurde.
Fig. 10 Durchschnittliche Wachstumsrate mit Rhizoctonia solani-infizierten Tabakpflanzen
   Darstellung der durchschnittlichen Wachstumsraten, die aus den in Fig. 9 gezeigten Einzelwerten errechnet wurden.
Fig. 11 Durchschnittliches Stengelwachstum Rhizoctonia solani-infizierten Tabakpflanzen
   Durchschnittliches Wachstumsverhalten von 10 unabhängigen wun1-PSI-transgenen Tabakpflanzen ("PSI") und 10 untransformierten Tabakpflanzen ("SRI") nach Infektion mit Rhizoctonia solani. Auf der Abzisse ist die Länge der Stengel (bis zum Vegetationspunkt) angegeben, die Ordinate gibt die Anzahl der Tage nach der Infektion an.

### Beispiel 1

### A. Verwendete Materialien:

### Medien

Zur Anzucht von Bakterien wurden Medien verwendet, wie sie von Maniatis, T. et al in "Molecular cloning: a laboratory manual" Cold Spring Harbour Laboratory, Cold Spring Harbour, New York (1982) näher beschrieben werden.

### Pflanzenmedien

Die benutzten Medien leiten sich von dem von Murashige, T. et al in "A rapid method for rapid growth and bioassays with tobacco tissue cultures"; Physiol. Plant., 15:473-497 (1962) angegebenen Medien (MS) ab.

| | |
|---|---|
| 3MS | MS + 3 % Saccharose |
| 3MSC | MS + 3 % Saccharose, 500 µg/ml Claforan |
| 3MC15 | MS + 2 % Saccharose, 500 µg/ml Claforan, + 100 µg/ml Kanamycinsulfat |
| MSC16 | MS + 0,5 µg/ml BAP + 0,1 µg/ml NAA |
| | + 100 µg/ml Ranamycinsulfat |
| | + 500 µg/ml Claforan |

Für festes Medium wurden zusätzlich 8 g/l Bacto-Agar zugegeben.

### Stämme und Vektoren

| | | |
|---|---|---|
| E. coli-Stämme | | |
| | BMH 71-18 | delta(1ac-proAB), thi, supE; F'(laci^{q}, ZdeltaM15, proA⁺B⁺) |

Verwiesen wird auf : Messing, J. et al. "Plant Gene Structure" in: Kosuge, F., Meredith, C.P., Hollaender, A. (Eds.). Genetic engineering of plants. Plenum Press, New York: 211-227 (1983)

| | |
|---|---|
| Agrobakterien-Stämme | LBA 4404: (Hoekema et al., Nature 303: 179-180 (1983) |
| Plasmide | pUC8 (Vieira und Messing in "The puc plasmid, an M13mp7-derived system for insertion mutagenesis and sequencing with synthetic universal primers. Gene, 19:259-268 (1982). |
| : | pPR69 (ein Derivat des bin 19, vgl. "Bevan, M. Binary Agrobacterium vectors for plant transformation", Nucl.Acids,Res. 12: 8711-8721 (1984)). |
| Pflanzen | Hordeum vulgare L. cv. Piggy Nicotiana tabacum SRI |

### B. Angewandte Methoden:

Alle molekularbiologischen Standard-Methoden, wie z. B. Restriktionsanalyse, Plasmidisolierung, Minipräparation von Plasmid-DNA, Transformation von Bakterien u.s.w. wurden, sofern nicht anders angegeben, wie bei Maniatis et al., (1982) beschrieben, durchgeführt.

### Pflanzenmaterial

Reife Gerste-Samen (Hordeum vulgare L. cv. Piggy) wurden zu verschiedenen Zeiten nach der Anthese geerntet, in flüssigem Stickstoff eingefroren und bei -80°C gelagert.

### Isolierung und Reinigung von PSI-, CHI- und BGL-Protein

### PSI- und CHI-Protein:

10 kg reife Gerstesamen werden zu feinem Mehl verarbeitet (Partikelgröße: unter 0,5 mm im Durchmesser). Nach Zusatz von 100 Liter Extraktionspuffer (50 mM Phosphatpuffer, pH 6,5; 100 mM NaCl; 2,5 mM Ascorbinsäure; 2,5 mM EDTA; 3 mM betamercapto-EtOH) und 2-stündigem Rühren bei 4°C wird der Überstand abfiltriert. Dazu wird mit Hilfe einer Ultrazentifuge das Volumen des Überstands auf 6 Liter eingeengt (verwendete Filter: DDS-Membranen (Ultrafiltrations-Membranen aus Polysulphon), die alle Proteine kleiner als 20 kDa festhalten). Der Überstand wird nun mit 40-70 %igem (NH₄)₂SO₄ gefällt. Das erhaltene Pellet löst man in 80 mM PMSF und dialysiert gegen 2 mM Na-Phosphatpuffer, pH 6,5, dem 80 mM PMSF zugegeben wurde. Nun wird die Proteinlösung über eine Ionenaustauschchromatografie auf CM52 (Firma: Whatman) geladen und mit 50 mM Na-phosphat, das steigende NaCl-Konzentrationen enthält (0,05 bis 1,0 M NaCl bei mehr als 10 Elutionsschritten), eluiert.

### BGL-Protein:

Gerste-Samen wurden 12 Tage gekeimt, lyophilisiert und mit Extraktionspuffer (siehe oben) verarbeitet (1,6 kg Samen/25 Liter Extraktionspuffer). Nach einer 40 %igen (NH₄)₂SO₄-Fällung wurde der Überstand dialysiert und über eine CM52 und Mono-S Säule gereinigt. Isoliertes BGL-Protein wurde durch Western-Blots und N-terminale Sequenzierung bezüglich der Reinheit getestet.

### Herstellung von PSI-Antikörpern

Antikörper wurden gegen gereinigtes PSI-II-Protein in Kaninchen nach herkömmlichen Methoden hergestellt.

### Pilzwachstumstest mit gereinigtem Protein

Trichoderma reesei und Fusarium sporotrichioides (ATCC-Sammlung, Rockville) wurden auf Kartoffel-Dextrose-Agar (Firma Difco) bei 25°C angezogen. Sporen von 8 Tage alten Kulturen wurden nach der Methode von Broekaert, W.F. et al. "An automated quantitative assay for fungal growth inhibition. FEMS Micobiology Letters, (1990) geerntet und bei -20°C in 20 %igem Glycerol gelagert.
Im Rahmen des Pilzwachstumstests wurde eine Sporensuspension (10.000 Sporen/ml) mit 100 µl kartoffel-Dextrose-Lösung sowie 35 µl einer zu testenden Proteinfraktion versetzt und bei 25°C inkubiert. Wie von Broekaert et al. beschrieben, ist das Wachstum des Pilzes mit der Zunahme der optischen Dichte bei 540 nm linear korreliert. Proteinfraktionen mit Pilzwachstumsinhibierender Wirkung führen somit zu einem geringeren Anstieg der optischen Dichte als wirkungslose Proteinfraktionen.

### Isolierung der PSI-cDNA Klone aus Gerste

Aus reifen Gerste-Samen (Hordeum vulgare L cv. Piggy) wurde PolyA⁺-RNA isoliert und eine cDNA-Expressions-Bank in lambdagt-11 Phagen angelegt. Verwiesen wird auf Leah, R. und Mundy, J. "The biofunctional a-amylase/subtilisin inhibitor of barley: nucleotide sequence and patterns of seed-specific expression". Plant Mol. Biol. 12:673-682 (1989). Mit Hilfe von monospezifischen Antikörpern PSI (vgl. Mundy, J. et al "Differential synthesis in vitro of barley aleurone ans starchy endosperm proteins." Plant Physiol. 81:630-636 (1986) wurden PSIenthaltende cDNA-Klone identifiziert.

### Analyse der PSI-Nukleotidsequenz

PSI-positive lambda-gt11-Phagen wurden vereinzelt, subkloniert und nach der Dideoxysequenzierungsmethode von Sanger et al., "DNA sequencing with chain-terminating inhibitors." Proc. Natl. Acad. Sci USA, 74:5463-5467 (1977) sequenziert.

### DNA Transfer in Agrobakterien

### Transformation:

Die in E. coli klonierte DNA wurde nach der von Van Haute et al. in der Arbeit "Intergenic transfer and exchange recombination of restriction fragments cloned in pBR322: a novel strategy for reversed genetics of Ti-plasmids of Agrobacterium tumefaciens", EMBO J., 2:411-418 (1983) beschriebenen Methode durch Konjugation nach A. tumefaciens LBA4404 (vgl. Hoekema et al. "A binary plant vector strategy based on separation of virand T-region of A-tumefaciens", Nature 303: 179-180 (1983)) transferiert.

### DNA-Analyse

Die Überprüfung des DNA Transfers in das Agrobacterium erfolgte durch die Isolierung von Agrobakterien DNA nach der von Ebert et al. in "Identification of an essential upstream element in the nopalin synthase promoter by stable and transient assays." Proc.Natl.Acad.Sci USA 84: 5745-5749 (1987) beschriebenen Methode. Restriktionsspaltung der DNA, der Transfer auf Nitrocellulose und die Hybridisierung gegen die entsprechende radioaktive Sonde gaben Aufschluß über einen erfolgreichen DNA Transfer in Agrobacterium.

### Transformation von Tabakpflanzen mit Agrobakterien

### Anzucht von Agrobakterien:

Die zur Infektion benötigten Agrobakterien LBA4404 wurden in selektivem Antibiotika-Medium angezogen (vgl. Zambrisky et al. "Ti-Plasmid vector for the introduction of DNA into plant cells without alteration of their normal capacity". EMBO J., 1:147-152 (1983)), durch Zentifugation sedimentiert und in YEB-Medium ohne Antibiotika gewaschen (YEB = 0,5 % Fleisch-Extrakt; 0,2 % HefeExtrakt; 0,5 % Pepton; 0,5 % Saccharose; 2 mM MgSO₄). Nach erneuter Sedimentation und Aufnahme in 3MS-Medium konnten die Bakterien zur Infektion verwandt werden.

### Blattscheiben-Infektion:

Für die Blattscheiben-Infektion wurden sterile Blätter der Tabaklinien SRI verwendet. Etwa 1 cm große Blattstücke in die wie oben beschriebene Agrobakteriensuspension eingetaucht, mit anschließender Überführung auf 3MS-Medium. Nach 2-tägiger Inkubation bei 16 Stunden Licht und 25°C - 27°C wurden die Blattstücke auf MSC16-Medium überführt. Nach 4-6 Wochen erscheinende Sprosse wurden abgeschnitten und auf MSC15-Medium umgesetzt. Sprosse mit Wurzelbildung wurden weiter analysiert.

### DNA-Analyse von Pflanzen:

Das Pflanzenmaterial wird mit flüssigem Stickstoff gemörsert, mit 10 Volumen Extraktionspuffer (10 mM Tris-HCl (pH 8); 100 mM NaCl, 1 mM EDTA, Proteinase K; pankreatische RNase) versetzt und inkubiert, mit Phenol extrahiert und der Überstand mit EtOH gefällt. Der Restriktionsverdau der isolierten DNA, die gelelektrophoretische Auftrennung der DNA mit Agarose, der Transfer der DNA auf eine Nylonmembran ist bei Maniatis et al., (1982) beschrieben. Die Hybridisierung gegen radioaktiv markierte DNA-Proben erfolgte nach einem von Logemann et al. in der Arbeit "Improved method for the isolation of RNA from plant tissues", Anal. Biochem., 163:16-20 (1987) beschriebenen Verfahren.

### RNA-Analyse von Pflanzen

### Gerste-Pflanzen:

Die Isolierung von Gesamt-RNA sowie PolyA⁺RNA erfolgte nach Leah and Mundy et al., (1989). Die gelelektrophoretische Auftrennung mit Formaldehydgelen, der Transfer auf Nylonmembranen sowie die Hybridisierung gegen radioaktiv markierte DNA-Proben wurde nach Maniatis et al., (1982) durchgeführt.

### Transgene Tabak- und Kartoffel-Pflanzen:

Die Isolierung von Gesamt-RNA aus verschiedenen Organen, der Transfer auf Nylonmembranen sowie die Hybridisierung gegen radioaktiv markierte DNA-Proben erfolgte nach Logemann et al., (1987).

### Protein-Analyse von transgenen Pflanzen

Lyophilisiertes Blattmaterial wurde im Extraktionspuffer (10 mM Tris pH 8,0; 1 mM EDTA; 100 mM NaCl; 2 % SDS) gemörsert und die Proteinkonzentration auf 1 mg/ml eingestellt. Die gelelektrophoretische Auftrennung des Proteins erfolgte mit den Phast-Gel-System (Pharmacia), wobei 1 µg Protein pro Slot aufgetragen wurde. Die aufgetrennten Proteine wurden auf Nitrocellulose transferiert (Diffusionsblotten durch 20-minütiges Auflegen der Nitrocellulose auf das Proteingel bei 70°C) und durch die Verwendung von spezifischen Antikörpern analysiert (Western-Blot-Analyse nach dem Proto-Blot-System der Firma Promega).

### Infektion von transgenen Pflanzen mit Rhizoctonia solani

Der Pilz Rhizoctonia solani wird in einem Flüssigmedium (Kartoffel-Dextrose-Agar der Firma Difco) bei 28°C angezogen und nach 5-6 Tagen geerntet. Mittels eines Büchnertrichters und angeschlossener Saugflasche wird das Medium abgezogen. Das zurückbleibende Pilzmycel wird mit einem Skalpell in möglichst kleine Fragmente zerlegt. Die gewünschte Menge Pilzmycel wird eingewogen und mit 5 Litern steriler Einheitserde gründlich vermischt. Diese Erde wird in einer Schale verteilt und mit den zu testenden Pflanzen bepflanzt. Das Wachstum der Pflanzen wird alle 24 Stunden durch die Bestimmung der Sproßlänge (Abstand Boden-Vegetationspunkt) ermittelt.

### Ergebnisse

### Isolierung und Reinigung von PSI-, CHI- und BGL-Proteinen aus Gerste-Samen:

Die Isolierung von CHI- und PSI-Protein aus reifen Gerste-Samen (Hordeum vulgare L. cv. Piggy) ist in "Methoden" beschrieben. Die im Rahmen der diversen Reinigungsschritte entstehenden Proteinfraktionen wurden auf einem denaturierenden Acrylamid-Gel aufgetragen und CHI- bzw. PSI-Proteine durch silbermarkierte CHI- bzw. PSI-Antikörper dargestellt (Fig. 1). CHI- bzw. PSI-Protein ist nach 40 %iger und 70 %iger (NH₄)₂SO₄-Fällung (Reihe 2,7), nach der anschließenden Auftrennung über Whatman CM52 (Reihe 3,4,8) und nach der folgenden Reinigung über eine Mono-S-Säule (Reihe 5,9,10,11) nachweisbar. Die Reihen 9, 10, 11 der Fig. 1 zeigen, daß drei verschiedene PSI-Isoformen (PSI I, II, III) isoliert werden sind, die sich durch ihr unterschiedliches Laufverhalten in der CM 52 Säule auszeichnen.

Die spezifische Aktivität von gereinigtem CHI-Protein wurde nach Molano et al., "A rapid and sensitive assay for chitinase using tritiated chitin", Anal. Biochem. 88:648-656 (1977) bestimmt und beträgt 22 mg Diacetylchitobiose/Minute/mg Protein.

Das gereinigte PSI-Protein zeigt folgende Aktivität: 3-30 ng PSI sind in der Lage, 50 % der RNA-Translation in Retikulozyten-Lysaten zu inhibieren.

BGL-Protein wurde aus 12 Tage alten Gerste-Sämlingen durch (NH₄)₂SO₄-Fällung, Auftrennung über CM52 und einer Mono-S-Säule gereinigt (siehe " Methoden") und mit Hilfe von BGL-Antikörpern nachgewiesen (Fig. 1 Reihe 13-17). Die spezifische Aktivität von gereinigtem BGL-Protein beträgt 25 mg Glukose-Äquivalente/Minute/mg Enzym.

### Pilzwachstumstest mit gereinigten Proteinen:

Wie in "Methoden" beschrieben, werden verschiedene Pilzarten auf je 135 µl Pilzmedium in Mikrotiterplatten (96 Löcher/Platte) angezogen und deren Wachstum fotometrisch verfolgt. Durch die Zugabe verschiedener Proteine kann deren Einfluß auf das Pilzwachstum analysiert werden.

In Fig. 2A ist das Wachstumsverhalten des Pilzes Trichoderma reesei dargestellt. Die Verwendung von 1,5 µg PSI/Loch inhibiert das Pilzwachstum nur um 20 %. Dagegen wird das Wachstum um mehr als 95 % inhibiert, wenn man je 0,25 µg der Proteine PSI, CHI und BGL miteinander kombiniert. Eine 95 %ige Inhibition erhält man auch durch die Kombination PSI/BGL (je 1,0 µg Protein) bzw. durch die Kombination PSI/CHI (je 1,5 µg Protein).

Auch das Wachstum von Fusarium sporotrichioides wird zu 95 % inhibiert, wenn man je 0,25 µg Protein von PSI, CHI und BGL kombiniert (Fig. 2B). Die Kombination PSI/CHI (je 1,0 µg Protein) inhibiert den Pilz in gleichen Maße. Die Verwendung von je 1,5 µg PSI oder CHI oder BGL alleine führt dagegen zu signifikant geringeren Inhibitionen. Einen geringeren Effekt hat auch die Kombination PSI/BGL.

Die in Fig. 2A und 2B ermittelten Daten zeigen, daß die Verwendung von PSI allein einen relativ geringen Hemmeffekt gegen die hier verwendeten Pilze hat. Die Kombination mit der Chitinase CHI (Fig. 2A,B) oder mit der Glucanase BGL (Fig. 2A) verstärkt den Hemmeffekt jedoch erheblich.

### Isolierung und Sequenzierung eines PSI-cDNA-Klons:

Aus reifen Gerste-Samen der Sorte Hordeum vulgare L. cv. Piggy wurde PolyA⁺-RNA isoliert, in cDNA umgeschrieben und in lambdagt-11 Phagen kloniert (siehe "Material und Methoden"). Ein nahezu vollständiger PSI-cDNA-Klon konnte durch die Verwendung von PSI-Antikörpern identifiziert werden. Die Sequenzierung des PSI-cDNA Klons ergab folgende Daten (Fig. 3A):
- Der PSI-Klon hat eine Länge von 1087 bp.
- Das GC-reiche offene Leseraster codiert für ein Protein mit einem Molekulargewicht von 29.976 Dalton.
- Das PSI-Protein enthält kein Signalpeptid.
- Das PSI Protein beginnt mit der Aminosäure Methionin und stimmt damit mit dem Proteinbeginn des natürlich vorkommenden PSI-Proteins überein. Daher ist zu erwarten, daß das PSI-Protein ein cytosolisches Protein ist.

Die in Fig. 3B gezeigte DNA-Sequenz ist unvollständig, zeigt jedoch große Homologien zum cDNA-Klon gemäß Fig. 3A und ist zur Lösung der gestellten Aufgabe geeignet, wenn die gezeigte DNA-Sequenz im 5'-Bereich durch einen entsprechenden cDNA-Klon komplettiert wird.

### Nachweis von PSI-Genen im Gerste-Genom:

Die Verwendung des PSI-cDNA-Klons als radioaktive Sonde ermöglicht eine Analyse des Gerste-Genoms bezüglich der Organisation und Anzahl von PSI-Genen. DNA aus Gerste Embryos wurde isoliert, mit verschiedenen Restriktionsenzymen geschnitten und gegen die PSI-cDNA Probe hybridisiert. Wie in Fig. 4 gezeigt, hybridisieren hauptsächlich drei Fragmente gegen PSI; es ist somit mit drei PSI-Genen pro haploidem Genom zu rechnen.

### Nachweis von PSI-mRNA in Gerste-Samen:

Die Expression von PSI-mRNA in verschiedenen Organen von Gerste-Pflanzen sowie im Rahmen der Samenentwicklung wurde mit Hilfe von Northern-Blot-Analysen bestimmt (siehe "Material und Methoden"). Wie Fig. 5 zeigt, ist in Gerste-Wurzeln, -Stengeln und -Blättern sowie in der Aleuronschicht von Gerste-Samen keine PSI-mRNA vorhanden. Hingegen findet man große Mengen an PSI-mRNA im stärkehaltigen Endosperm aus Samen, sofern sich der Samen in einem späten Entwicklungsstadium befindet. In jungem Endosperm ist keine PSI-mRNA vorhanden.

### Fusion eines PSI-Gens mit dem wunl-Promotor und Transfer in Tabakpflanzen:

Wie in Fig. 6 beschrieben, wurde der PSI-cDNA-Klon mit dem wundund pathogen-induzierbaren Promotor wun1 transkriptionell fusioniert.

Der wunl-Promotor (1022 bp) sowie 179 bp des 5'-untranslatierten Bereichs von wun1 mit dem über EcoRI aus der cDNA-Bank isolierten PSI-cDNA-Klon fusioniert. Das PSI-Gen verfügt über ein eigenes Polyadenylierungssignal, dessen Funktionalität in dicotylen Pflanzen bisher jedoch nur vermutet werden kann. Zwei zusätzliche Elemente wurden hinter das PSI-Gen kloniert, um die Stabilität der PSI-mRNA zu erhöhen. 3' vom PSI-Gen wurde eine ca. 500 bp lange Teilsequenz des CAT-Gens (CAT-Chloramphenicolacetyltransferase) fusioniert. 3' der CAT-Teilsequenz wurde das Polyadenylierungssignal des 35S-Gens des Blumenkohlmosaikvirus als Terminierungssignal verwendet, dessen Funktionalität in dicotylen Pflanzen bekannt ist.

Das chimäre Gen wun1-PSI wurde über die HindIII-Schnittstellen in den binären Vektor pPR69 kloniert, dessen Bezeichnung nun "wunl-PSI in pPR69" ist. Wun1-PSI in pPR69 wurde mittels des Agrobacterium tumefaciens Transformationssystems in Tabakpflanzen transformiert und Kanamycin-resistente Tabakpflanzen regeneriert.

### Nachweis des wunl-PSI Gens in transgenen Pflanzen:

Mit Hilfe der Southern-Blot-Analyse wurden die wunl-PSI transgenen Tabakpflanzen bezüglich der korrekten Integration von wun1-PSI in das Tabakgenom untersucht. Wie Fig. 7 zeigt, entspricht die Größe der hybridisierenden DNA-Bande aus den transgenen Tabakpflanzen der Größe des eingebrachten Fragments. Daher ist eine korrekte Integration von wunl-PSI in das Tabakgenom wahrscheinlich.

### Nachweise von PSI-Genexpressionen in transgenen Tabakpflanzen:

Je 50 µg Gesamt-RNA wurden aus pilzinfizierten wun1-PSItransgenen Tabakblättern isoliert und auf das Vorhandensein von PSI-mRNA untersucht (Fig. 8). In untransformierten Tabakblättern (K) wurde keine PSI-mRNA nachgewiesen, unabhängig davon, ob die Blätter unverwundet, verwundet oder pilzinfiziert waren. In Tabakblättern pilzinfizierter, transgener Pflanzen ist eine mit PSI hybridisierende RNA-Bande erkennbar. Der Nachweis von PSI-Proteinen in transgenen Tabakpflanzen erfolgte durch die Western-Blot-Methode.

### Infektion von wun1-PSI transgenen Tabakpflanzen mit Rhizoctonia solani:

Verschiedene unabhängige wunl-PSI transgene Pflanzen wurden als ca. 10 cm große Pflanzen in Erde gesetzt, die mit Rhizoctonia solani (5 g/5 Liter Erde) infiziert worden war. Durch die tägliche Messung der Pflanzengröße (Entfernung: Pflanzenvegetationspunkt-Erde) wurde das Wachstum der Pflanzen für die nächsten ca. 2 Wochen festgehalten. Als Kontrollpflanzen dienten untransformierte Tabakpflanzen, die
1. ebenfalls in mit Rhizoctonia solani (5g/5 Liter Erde) infizierte Erde gepflanzt wurden;
2. in Erde ohne Zusatz von Rhizoctonia solani gepflanzt wurden.

Fig. 9 zeigt das Wachstumsverhalten der einzelnen Pflanzen unter den entsprechenden Bedingungen an. Die Wachstumsrate von untransformierten Tabakpflanzen ist in Rhizoctonia solanihaltigen Erde sehr niedrig. Wun1-PSI transgene Tabakpflanzen zeigen dagegen auf einem Rhizoctonia solani enthaltenden Boden eine wesentlich höhere Wachstumsrate.

Die Wachstumsrate ist nur geringfügig kleiner als die Rate von untransformierten Tabakpflanzen, die in pilzfreiem Boden angezogen worden sind.

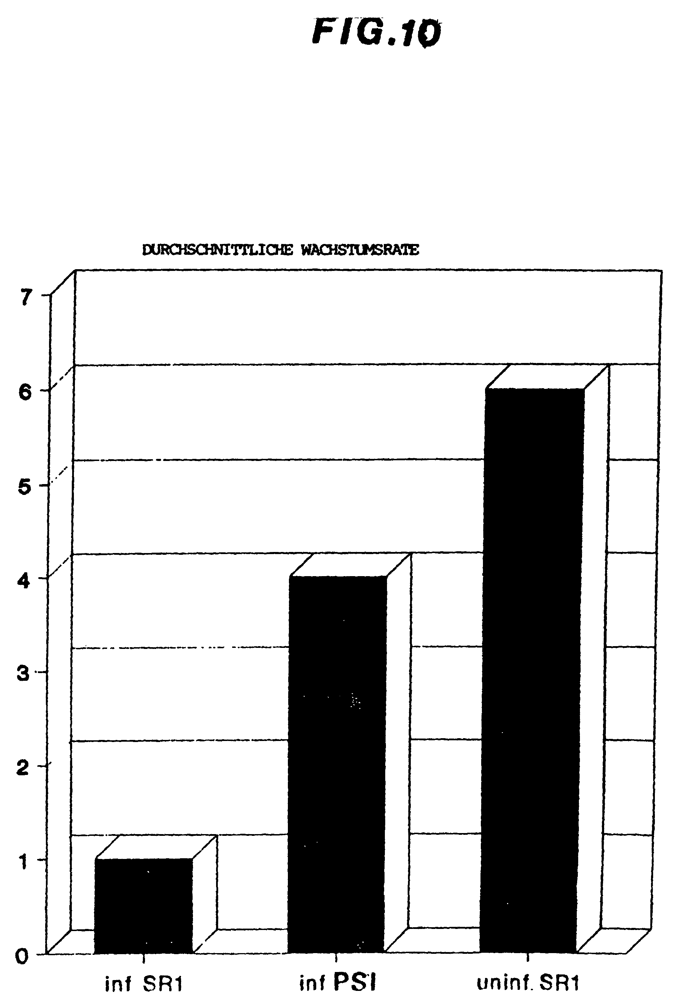

Fig. 10 beschreibt den statistischen Mittelwert aus den in Fig. 9 gezeigten Einzelwerten.

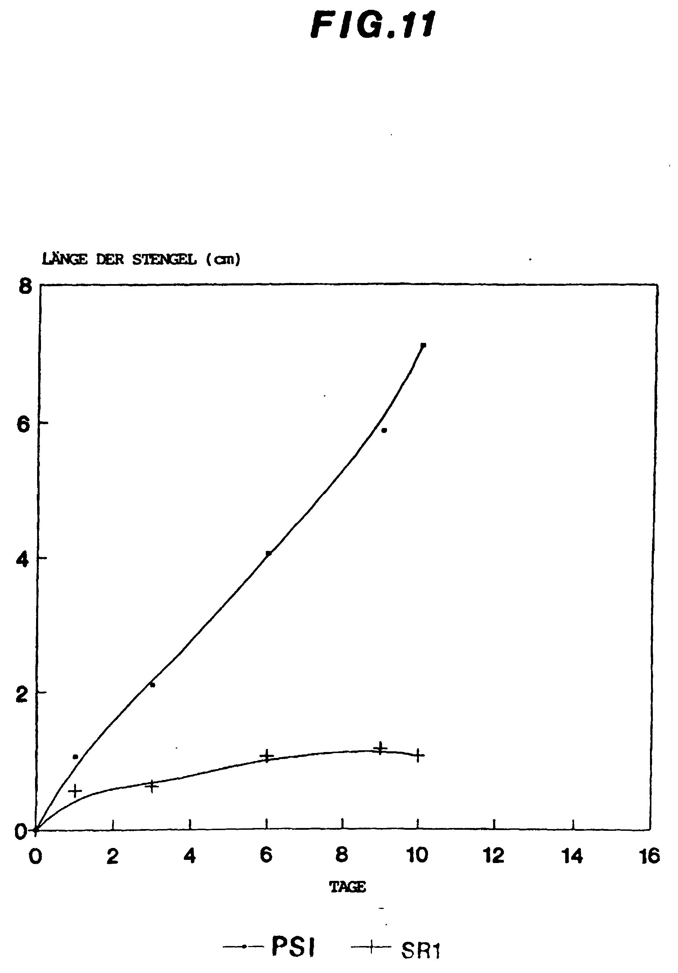

Fig. 11 zeigt das durchschnittliche Wachstum der Tabakpflanzen unter den verschiedenen Bedingungen an. In einem Zeitraum von ca. 10 Tagen wachsen untransformierte Tabakpflanzen auf Rhizoctonia solani-haltigem Boden etwa 1 cm. Wun1-PSI transgene Tabakpflanzen wachsen auf Rhizoctonia solani-haltigem Boden ca. 4 cm/10 Tage. Untransformierte Tabakpflanzen, die auf Rhizoctonia solani-freiem Boden gehalten werden, wachsen etwa 6 cm in ca. 10 Tagen.

### Beispiel 2

### Isolierung und Reinigung des PSI-Proteins aus bakteriellen Überproduzenten

Eine Beispiel für ein geeignetes Plasmid zur bakteriellen Überproduktion von PSI-Proteinen ist das Plasmid pKK223-3 (Hersteller: Fa. Pharmazia).

Ein IPTG (isopropyl-β-D-thiogalactosid) induzierbarer (tac-Promotor ermöglicht beispielsweise die Produktion des PSI-Proteins. Verschiedene Restriktionsstellen unmittelbar hinter dem tac-Promotor erlauben die transkriptionelle Fusion des PSI-Gens mit dem tac-Promotor. Ein starker ribosomaler Terminator (rrn) bewirkt einen definierten Abbruch der Transkription.

Das PSI-Gen wurde über die EcoRI-Schnittstelle in 5'3'-Orientierung in die EcoRI-Schnittstelle von pKK233-3 kloniert und in JM105-Bakterien transformiert. Diese Bakterien wurden in 100 ml LB-Medium (50 µg/ml Ampecillin) bei 37°C unter kräftigem Schütteln bis zu einer O.D.₅₅₀=0,4 herangezogen und anschließend mit IPTG (2,5 mM Endkonzentration) versetzt.
Es folgte eine weitere Inkubation über 4 Stunden bei 37°C. Anschließend wurde die 100 ml Bakterienkultur für 15 Minuten bei 2500 rpm (Christ-Zentrifuge, 4°C) abzentrifugiert und das Bakterienpellet in 50 mM Tris pH 8,0 aufgenommen. Die Suspension wurde mit Ultraschall (mehrmals 2 Minuten mit 60%-Pulsen) sonifiziert, bis die Viskosität deutlich nachließ.

Analog der in "Methoden" vorliegenden Beschreibung zur "Isolierung und Reinigung von PSI-Protein" wurde das PSI-Protein nun mit 40-70 %igem (NH₄)₂SO₄ gefällt und durch Ionenaustauschchromatografie z. B. CM52 gereinigt.

Das gereinigte und sterilfiltrierte Protein eignete sich zur Herstellung von Infusionslösungen für die therapeutische Verwendung bei Menschen und Tieren.

## Patentansprüche

1. Eine DNA, die für ein Protein kodiert, das die folgende Aminosäuresequenz umfaßt:

M A A K M A
-1 1

K N V D K P L F T A T F N V Q A S S A D
10 20

Y A T F I A G I R N K L R N P A H F S H
30 40

N R P V L P P V E P N V P P S R W F H V
50 60

V L K A S P T S A G L T L A I R A D N I
70 80

Y L E G F K S S D G T W W E L T P G L I
90 100

P G A T Y V G F G G T Y R D L L G D T D
110 120

K L T N V A L G R Q Q L A D A V T A L H
130 140

G R T K A D K P S G P K Q Q Q A R E A V
150 160

T T L L L M V N E A T R F Q T V S G F V
170 180

A G L L H P K A V E K K S G K I G N E M
190 200

K A Q V N G W Q D L S A A L L K T D V K
210 220

P P P G K S P A K F A P I E K M G V R T
230 240

A V Q A A N T L G I L L F V E V P G G L
250 260

T V A K A L E L F H A S G G K *

2. Die DNA nach Anspruch 1, umfassend die Nukleotidsequenz des offenen Leserasters von:

CTTAATAGCACATCTTGTCCGTCTTAGCTTTGCATTACATCCATGGCGGCAAAGATGGCG 60
*                                        M  A  A  K  M  A
                                        -1  1

AAGAACGTGGACAAGCCGCTCTTCACCGCGACGTTCAACGTCCAGGCCAGCTCCGCCGAC 120
K  N  V  D  K  P  L  F  T  A  T  F  N  V  Q  A  S  S  A  D
            10                            20

TACGCCACCTTCATCGCCGGCATCCGCAACAAGCTCCGCAACCCGGCGCACTTCTCCCAC 180
Y  A  T  F  I  A  G  I  R  N  K  L  R  N  P  A  H  F  S  H
            30                            40

AACCGCCCCGTGCTGCCGCCGGTCGAGCCCAACGTCCCGCCGAGCAGGTGGTTCCACGTC 240
N  R  P  V  L  P  P  V  E  P  N  V  P  P  S  R  W  F  H  V
            50                            60

GTGCTCAAGGCCTCGCCGACCAGCGCCGGGCTCACGCTGGCCATTCGGGCGGACAACATC 300
V  L  K  A  S  P  T  S  A  G  L  T  L  A  I  R  A  D  N  I
            70                            80

TACCTGGAGGGCTTCAAGAGCAGCGACGGCACCTGGTGGGAGCTCACCCCGGGCCTCATC 360
Y  L  E  G  F  K  S  S  D  G  T  W  W  E  L  T  P  G  L  I
            90                           100

CCCGGCGCCACCTACGTCGGGTTCGGCGGCACCTACCGCGACCTCCTCGGCGACACCGAC 420
P  G  A  T  Y  V  G  F  G  G  T  Y  R  D  L  L  G  D  T  D
           110                           120

AAGCTGACCAACGTCGCTCTCGGCCGGCAGCAGCTGGCGGACGCGGTGACCGCCCTCCAC 480
K  L  T  N  V  A  L  G  R  Q  Q  L  A  D  A  V  T  A  L  H
           130                           140

GGGCGCACCAAGGCCGACAAGCCGTCCGGCCCGAAGCAGCAGCAGGCGAGGGAGGCGGTG 540
G R T K A D K P S G P K Q Q Q A R E A V
150 160

ACGACGCTGCTCCTCATGGTGAACGAGGCCACGCGGTTCCAGACGGTGTCTGGGTTCGTG 600
T T L L M V N E A T R F Q T V S G F V
170 180

GCCGGGTTGCTGCACCCCAAGGCGGTGGAGAAGAAGAGCGGGAAGATCGGCAATGAGATG 660
A G L L H P K A V E K K S G K I G N E M
190 200

AAGGCCCAGGTGAACGGGTGGCAGGACCTGTCCGCGGCGCTGCTGAAGACGGACGTGAAG 720
K A Q V N G W Q D L S A A L L K T D V K
210 220

CCTCCGCCGGGAAAGTCGCCAGCGAAGTTCGCGCCGATCGAGAAGATGGGCGTGAGGACG 780
P P P G K S P A K F A P I E K M G V R T
230 240

GCTGTACAGGCCGCCAACACGCTGGGGATCCTGCTGTTCGTGGAGGTGCCGGGTGGGTTG 840
A V Q A A N T L G I L L F V E V P G G L
250 260

ACGGTGGCCAAGGCGCTGGAGCTGTTCCATGCGAGTGGTGGAAATAGGTAGTTTTCCAG 900
T V A K A L E L F H A S G G K *

GTATACCTGCATGGGTAGTGTAAAAGTCGAATAAACATGTCACAGAGTGACGGACTGATA 960

TAAATAAATAAATAAACGTGTCACAGAGTTACATATAAACAAATAAATAAATAATTAAAA 1020

ATGTCCAGTTTA47 1078

3. Die DNA nach Anspruch 1, umfassend die folgende Nukleotidsequenz:

CTTAATAGCACATCTTGTCCGTCTTAGCTTTGCATTACATCCATGGCGGCAAAGATGGCG 60

AAGAACGTGGACAAGCCGCTCTTCACCGCGACGTTCAACGTCCAGGCCAGCTCCGCCGAC 120

TACGCCACCTTCATCGCCGGCATCCGCAACAAGCTCCGCAACCCGGCGCACTTCTCCCAC 180

AACCGCCCCGTGCTGCCGCCGGTCGAGCCCAACGTCCCGCCGAGCAGGTGGTTCCACGTC 240

GTGCTCAAGGCCTCGCCGACCAGCGCCGGGCTCACGCTGGCCATTCGGCGGACAACATC 300

TACCTGGAGGGCTTCAAGAGCAGCGACGGCACCTGGTGGAGCTCACCCCGGGCCTCATC 360

CCCGGCGCCACCTACGTCGGGTTCGGCGGCACCTACCGCGACCTCCTCGGCGACACCGAC 420

AAGCTGACCAACGTCGCTCTCGGCCGGCAGCAGCTGGCGGACGCGGTGACCGCCCTCCAC 480

GGGCGCACCAAGGCCGACAAGCCGTCCGGCCCGAAGCAGCAGCAGGCGAGGGAGGCGGTG 540

ACGACGCTGCTCCTCATGGTGAACGAGGCCACGCGGTTCCAGACGGTGTCTGGGTTCGTG 600

GCCGGGTTGCTGCACCCCAAGGCGGTGGAGAAGAAGAGCGGAAGATCGGCAATGAGATG 660

AAGGCCCAGGTGAACGGGTGGCAGGACCTGTCCGCGGCGCTGCTGAAGACGGACGTGAAG 720

CCTCCGCCGGGAAAGTCGCCAGCGAAGTTCGCGCCGATCGAGAAGATGGGCGTGAGGACG 780

GCTGTACAGGCCGCCAACACGCTGGGGATCCTGCTGTTCGTGGAGGTGCCGGGTGGGTTG 840

ACGGTGGCCAAGGCGCTGGAGCTGTTCCATGCGAGTGGTGGAAATAGGTAGTTTTCCAG 900

GTATACCTGCATGGGTAGTGTAAAAGTCGAATAAACATGTCACAGAGTGACGGACTGATA 960

TAAATAAATAAATAAACGTGTCACAGAGTTACATATAAACAAATAAATAAATAATTAAAA 1020

ATGTCCAGTTTA47 1078

4. Die DNA nach Anspruch 1 oder 2, die mit einem in Pflanzen aktiven Promotor fusioniert ist.

5. Die DNA nach Anspruch 4, wobei der Promotor ausgewählt ist aus: pathogeninduzierbare Promotoren, konstitutive Promotoren, entwicklungsspezifische Promotoren, organspezifische Promotoren, induzierbare Promotoren, oder wunl Promotor.

6. Ein DNA-Expressionsvektor, umfassend die DNA nach einem der Ansprüche 1 bis 5.

7. Ein Protein, umfassend die Aminosäuresequenz nach:

M A A K M A
-1 1
K N V D K P L F T A T F N V Q A S S A D
10 20
Y A T F I A G I R N K L R N P A H F S H
30 40
N R P V L P P V E P N V P P S R W F H V
50 60
V L K A S P T S A G L T L A I R A D N I
70 80
Y L E G F K S S D G T W W E L T P G L I
90 100

P G A T Y V G F G G T Y R D L L G D T D
110 120
K L T N V A L G R Q Q L A D A V T A L H
130 140
G R T K A D K P S G P K Q Q Q A R E A V
150 160
T T L L L M V N E A T R F Q T V S G F V
170 180
A G L L H P K A V E K K S G K I G N E M
190 200
K A Q V N G W Q D L S A A L L K T D V K
210 220
P P P G K S P A K F A P I E K M G V R T
230 240
A V Q A A N T L G I L L F V E V P G G L
250 260
T V A K A L E L F H A S G G K

8. Verwendung des Proteins nach Anspruch 7 zur Inhibierung des Wachstums von Pflanzenpathogenen.

9. Verwendung des Proteins nach Anspruch 7, um pathogeninhibierende Eigenshaften in einer Pflanze zu erhalten.

10. Verwendung nach Anspruch 8 oder 9, worin das Pathogen ein Pilz ist.

11. Verwendung nach Anspruch 10, worin der Pilz ausgewählt ist aus: Trichoderma reesei, Rhizoctonia solani oder Fusarium sporotrichioides.

12. Verfahren zur Inhibierung des Wachstums eines für Pflanzen pathogenen Pilzes, umfassend das Aufbringen eines Proteins nach Anspruch 7 auf den Pilz.

13. Verfahren nach Anspruch 12, worin auch eine Glucanase BGL und/oder eine Chitinase CHI aus Gerstensamen auf den Pilz aufgebracht werden.

14. Verfahren nach Anspruch 12 oder 13, worin der Auftrag auf den Pilz durch die Expression in einer transgenen Pflanze erfolgt.

15. Ein Verfahren zur Erzeugung von pathogen-resistenten Pflanzen, umfassend die Transformierung einer Pflanze mit der DNA nach Anspruch 4 oder einer ähnlichen, davon durch Nukleotid-Substitution abgeleiteten DNA-Sequenz, die für das Protein nach Anspruch 7 kodiert.

16. Verfahren nach Anspruch 15, worin die Pflanze mit dem offenen Leseraster der folgenden DNA Sequenz transformiert wurde, die im 5'-Bereich mit einem entsprechenden cDNA-Klon komplettiert worden ist:

GCGGTGACGACGCTGCTCCTCATGGTGAACGAGGCCACGCGGTTCCAGACGGTGTCGGGG 60
A V T T L L M V N E A T R F Q T V S G
170 180

TTCGTGGCCGGGCTGCTGCACCCCAAGGCGGTGGAGAAGAAGAGCGGGAAGATCGGCAAT 120
F V A G L L H P K A V E K K S G K I G N
190 200

GAGATGAAGGCCCAGGTGAACGGGTGGCAGGACCTGTCCGCGGCGCTGCTGAAGACGGAC 180
E M K A Q V N G W Q D L S A A L L K T D
210 220

GTGAAGCCCCCGCCGGGAAAGTCGCCAGCGAAGTTCACGCCGATCGAGAAGATGGGCGTG 240
V K P P P G K S P A K F T P I E K M G V
230 240

AGGACTGCTGAGCAGGCTGCGGCTACTTTGGGGATCCTGCTGTTCGTTGAGGTGCCGGGT 300
R T A E Q A A A T L G I L L F V E V P G
250 260

GGGTTGACGGTGGCCAAGGCGCTGGAGCTGTTTCATGCGAGTGGTGGAAATAGGTAGTT 360
G L T V A K A L E L F H A S G G K *
270 280

TTGCAGGTATACCTGCATGGGTAAATGTAAAAGTCGAATAAAAATGTCACAGAGTGACGG 420

ACTGATATAAATAAATTAATAAACATGTCATCATGAGTGACAGACTGATATAAATAAATA20 499

17. Verfahren nach Anspruch 15 oder 16, worin das Pathogen ein Pilz ist.

18. Verfahren nach Anspruch 17, worin der Pilz ausgewählt ist aus: Trichoderma reesei, Rhizoctonia solani oder Fusarium sporotrichioides.

19. Eine Pflanzenzelle, die das Wachstum eines Pilz-Pathogens inhibieren kann, die so transformiert wurde, daß sie in ihrem Genom die DNA nach Anspruch 4 oder eine ähnliche, davon durch Nukleotid-Substitution abgeleitete DNA-Sequenz enthält und die für das Protein nach Anspruch 7 kodiert; wobei natürlich vorkommende Pflanzenzellen ausgenommen sind.

20. Pflanzenzelle nach Anspruch 19, worin die Pflanzenzelle mit einer DNA, umfassend das offene Leseraster der folgenden DNA Sequenz,transformiert wurde, welche im 5'-Bereich durch einen entsprechenden cDNA-Klon komplettiert worden ist:

GCGGTGACGACGCTGCTCCTCATGGTGAACGAGGCCACGCGGTTCCAGACGGTGTCGGGG 60
A V T T L L L M V N E A T R F Q T V S G
170 180

TTCGTGGCCGGGCTGCTGCACCCCAAGGCGGTGGAGAAGAAGAGCGGGAAGATCGGCAAT 120
F V A G L L H P K A V E K K S G K I G N
190 200

GAGATGAAGGCCCAGGTGAACGGGTGGCAGGACCTGTCCGCGGCGCTGCTGAAGACGGAC 180
E M K A Q V N G W Q D L S A A L L K T D
210 220

GTGAAGCCCCCGCCGGGAAAGTCGCCAGCGAAGTTCACGCCGATCGAGAAGATGGGCGTG 240
V K P P P G K S P A K F T P I E K M G V
230 240

AGGACTGCTGAGCAGGCTGCGGCTACTTTGGGGATCCTGCTGTTCGTTGAGGTGCCGGGT 300
R T A E Q A A A T L G I L L F V E V P G
250 260

GGGTTGACGGTGGCCAAGGCGCTGGAGCTGTTTCATGCGAGTGGTGGGAAATAGGTAGTT 360
G L T V A K A L E L F H A S G G K *
270 280

TTGCAGGTATACCTGCATGGGTAAATGTAAAAGTCGAATAAAAATGTCACAGAGTGACGG 420

ACTGATATAAATAAATTAATAAACATGTCATCATGAGTGACAGACTGATATAAATAAATA20 499

21. Pflanzenzelle nach Anspruch 19 oder 20, welche eine Zelle einer monocotylen Pflanze, einer Getreidepflanze, einer Maispflanze, einer dicotylen Pflanze, einer Solanacee oder Cucurbitacee ist.

22. Pflanzen oder Pflanzenteile, die im wesentlichen aus den Pflanzenzellen nach einem der Ansprüche 19 oder 20 bestehen.

23. Pflanze nach Anspruch 22, die ausgewählt ist aus einer monocotylen Pflanze, einer Getreidepflanze, einer Maispflanze, einer dicotylen Pflanze, einer Solanacee oder einer Cucurbitacee.

## Claims

1. A DNA coding for a protein comprising the following amino acid sequence:

M A A K M A
-1 1

K N V D K P L F T A T F N V Q A S S A D
10 20

Y A T F I A G I R N K L R N P A H F S H
30 40

N R P V L P P V E P N V P P S R W F H V
50 60

V L K A S P T S A G L T L A I R A D N I
70 80

Y L E G F K S S D G T W W E L T P G L I
90 100

P G A T Y V G F G G T Y R D L L G D T D
110 120

K L T N V A L G R Q Q L A D A V T A L H
130 140

G R T K A D K P S G P K Q Q Q A R E A V
150 160
T T L L L M V N E A T R F Q T V S G F V
170 180
A G L L H P K A V E K K S G K I G N E M
190 200
K A Q V N G W Q D L S A A L L K T D V K
210 220
P P P G K S P A K F A P I E K M G V R T
230 240
A V Q A A N T L G I L L F V E V P G G L
250 260
T V A K A L E L F H A S G G K *

2. The DNA according to claim 1, comprising the nucleotide sequence of the open reading frame of:

CTTAATAGCACATCTTGTCCGTCTTAGCTTTGCATTACATCCATGGCGGCAAAGATGGCG 60
*                                    M  A  A  K  M  A
-1 1

AAGAACGTGGACAAGCCGCTCTTCACCGCGACGTTCAACGTCCAGGCCAGCTCCGCCGAC 120
K  N  V  D  K  P  L  F  T  A  T  F  N  V  Q  A  S  S  A  D
10                            20

TACGCCACCTTCATCGCCGGCATCCGCAACAAGCTCCGCAACCCGGCGCACTTCTCCCAC 180
Y  A  T  F  I  A  G  I  R  N  K  L  R  N  P  A  H  F  S  H
30                            40

AACCGCCCCGTGCTGCCGCCGGTCGAGCCCAACGTCCCGCCGAGCAGGTGGTTCCACGTC 240
N  R  P  V  L  P  P  V  E  P  N  V  P  P  S  R  W  F  H  V
50                            60

GTGCTCAAGGCCTCGCCGACCAGCGCCGGGCTCACGCTGGCCATTCGGGCGGACAACATC 300
V  L  K  A  S  P  T  S  A  G  L  T  L  A  I  R  A  D  N  I
70                            80

TACCTGGAGGGCTTCAAGAGCAGCGACGGCACCTGGTGGGAGCTCACCCCGGGCCTCATC 360
Y  L  E  G  F  K  S  S  D  G  T  W  W  E  L  T  P  G  L  I
90                            100

CCCGGCGCCACCTACGTCGGGTTCGGCGGCACCTACCGCGACCTCCTCGGCGACACCGAC 420
P  G  A  T  Y  V  G  F  G  G  T  Y  R  D  L  L  G  D  T  D
110                           120

AAGCTGACCAACGTCGCTCTCGGCCGGCAGCAGCTGGCGGACGCGGTGACCGCCCTCCAC 480
K  L  T  N  V  A  L  G  R  Q  Q  L  A  D  A  V  T  A  L  H
130                           140

GGGCGCACCAAGGCCGACAAGCCGTCCGGCCCGAAGCAGCAGCAGGCGAGGGAGGCGGTG 540
G R T K A D K P S G P K Q Q Q A R E A V
150 160

ACGACGCTGCTCCTCATGGTGAACGAGGCCACGCGGTTCCAGACGGTGTCTGGGTTCGTG 600
T T L L L M V N E A T R F Q T V S G F V
170 180

GCCGGGTTGCTGCACCCCAAGGCGGTGGAGAAGAAGAGCGGGAAGATCGGCAATGAGATG 660
A G L L H P K A V E K K S G K I G N E M
190 200

AAGGCCCAGGTGAACGGGTGGCAGGACCTGTCCGCGGCGCTGCTGAAGACGGACGTGAAG 720
K A Q V N G W Q D L S A A L L K T D V K
210 220

CCTCCGCCGGGAAAGTCGCCAGCGAAGTTCGCGCCGATCGAGAAGATGGGCGTGAGGACG 780
P P P G K S P A K F A P I E K M G V R T
230 240

GCTGTACAGGCCGCCAACACGCTGGGGATCCTGCTGTTCGTGGAGGTGCCGGGTGGGTTG 840
A V Q A A N T L G I L L F V E V P G G L
250 260

ACGGTGGCCAAGGCGCTGGAGCTGTTCCATGCGAGTGGTGGAAAATAGGTAGTTTTCCAG 900
T V A K A L E L F H A S G G K *

GTATACCTGCATGGGTAGTGTAAAAGTCGAATAAACATGTCACAGAGTGACGGACTGATA 960

TAAATAAATAAATAAACGTGTCACAGAGTTACATATAAACAAATAAATAAATAATTAAAA 1020

ATGTCCAGTTTA47 .. 1078

3. The DNA according to claim 1, comprising the following nucleotide sequence:

CTTAATAGCACATCTTGTCCGTCTTAGCTTTGCATTACATCCATGGCGGCAAAGATGGCG 60

AAGAACGTGGACAAGCCGCTCTTCACCGCGACGTTCAACGTCCAGGCCAGCTCCGCCGAC 120

TACGCCACCTTCATCGCCGGCATCCGCAACAAGCTCCGCAACCCGGCGCACTTCTCCCAC 180

AACCGCCCCGTGCTGCCGCCGGTCGAGCCCAACGTCCCGCCGAGCAGGTGGTTCCACGTC 240

GTGCTCAAGGCCTCGCCGACCAGCGCCGGGCTCACGCTGGCCATTCGGCGGACAACATC 300

TACCTGGAGGGCTTCAAGAGCAGCGACGGCACCTGGTGGAGCTCACCCGGGCCTCATC 360

CCCGGCGCCACCTACGTCGGGTTCGGCGGCACCTACCGCGACCTCCTCGGCGACACCGAC 420

AAGCTGACCAACGTCGCTCTCGGCCGGCAGCAGCTGGCGGACGCGGTGACCGCCCTCCAC 480

GGGCGCACCAAGGCCGACAAGCCGTCCGGCCCGAAGCAGCAGCAGGCGAGGGAGGCGGTG 540

ACGACGCTGCTCCTCATGGTGAACGAGGCCACGCGGTTCCAGACGGTGTCTGGGTTCGTG 600

GCCGGGTTGCTGCACCCCAAGGCGGTGGAGAAGAAGAGCGGAAGATCGGCAATGAGATG 660

AAGGCCCAGGTGAACGGGTGGCAGGACCTGTCCGCGGCGCTGCTGAAGACGGACGTGAAG 720

CCTCCGCCGGGAAAGTCGCCAGCGAAGTTCGCGCCGATCGAGAAGATGGGCGTGAGGACG 780

GCTGTACAGGCCGCCAACACGCTGGGGATCCTGCTGTTCGTGGAGGTGCCGGGTGGGTTG 840

ACGGTGGCCAAGGCGCTGGAGCTGTTCCATGCGAGTGGTGGAAATAGGTAGTTTTCCAG 900

GTATACCTGCATGGGTAGTGTAAAAGTCGAATAAACATGTCACAGAGTGACGGACTGATA 960

TAAATAAATAAATAAACGTGTCACAGAGTTACATATAAACAAATAAATAAATAATTAAAA 1020

ATGTCCAGTTTA47 1078

4. The DNA according to claim 1 or 2, fused with a promoter active in plants.

5. The DNA according to claim 4, wherein the promoter is selected from: pathogen-inducible promoters, constitutive promoters, development-specific promoters, organ-specific promoters, inducible promoters or the wun1-promoter.

6. A DNA expression vector comprising the DNA according to one according to claims 1 to 5.

7. A protein comprising the amino acid sequence of:

M A A K M A
-1 1

K N V D K P L F T A T F N V Q A S S A D
10 20

Y A T F I A G I R N K L R N P A H F S H
30 40

N R P V L P P V E P N V P P S R W F H V
50 60

V L K A S P T S A G L T L A I R A D N I
70 80

Y L E G F K S S D G T W W E L T P G L I
90 100

P G A T Y V G F G G T Y R D L L G D T D
110 120

K L T N V A L G R Q Q L A D A V T A L H
130 140

G R T K A D K P S G P K Q Q Q A R E A V
150 160

T T L L L M V N E A T R F Q T V S G F V
170 180

A G L L H P K A V E K K S G K I G N E M
190 200

K A Q V N G W Q D L S A A L L K T D V K
210 220

P P P G K S P A K F A P I E K M G V R T
230 240

A V Q A A N T L G I L L F V E V P G G L
250 260

T V A K A L E L F H A S G G K

8. Use of the protein according to claim 7 for inhibiting the growth of plant pathogens.

9. Use of the protein according to claim 7 to maintain pathogen-inhibiting properties in a plant.

10. Use according to claim 8 or 9, wherein the pathogen is a fungus.

11. Use according to claim 10, wherein the fungus is selected from: Trichoderma reesei, Rhizoctonia solani or Fusarium sporotrichioides.

12. Method for inhibiting the growth of a plant-pathogenic fungus, comprising applying a protein according to claim 7 to the fungus.

13. Method according to claim 12, wherein in addition a glucanase BGL and/or a chitinase CHI from barley seeds is applied to the fungus.

14. Method according to claim 12 or 13, wherein the application to the fungus occurs by expression in a transgenic plant.

15. A method for producing pathogen-resistant plants, comprising the transformation of a plant with the DNA according to claim 4 or a similar DNA sequence derived therefrom by nucleotide substitution, coding for the protein according to claim 7.

16. Method according to claim 15, wherein the plant has been transformed with the open reading frame of the following DNA sequence, which has been completed in the 5'-region by a corresponding cDNA clone:

GCGGTGACGACGCTGCTCCTCATGGTGAACGAGGCCACGCGGTTCCAGACGGTGTCGGGG 60
A V T T L L L M V N E A T R F Q T V S G
170 180

TTCGTGGCCGGGCTGCTGCACCCCAAGGCGGTGGAGAAGAAGAGCGGGAAGATCGGCAAT 120
F V A G L L H P K A V E K K S G K I G N
190 200

GAGATGAAGGCCCAGGTGAACGGGTGGCAGGACCTGTCCGCGGCGCTGCTGAAGACGGAC 180
E M K A Q V N G W Q D L S A A L L K T D
210 220

GTGAAGCCCCCGCCGGGAAAGTCGCCAGCGAAGTTCACGCCGATCGAGAAGATGGGCGTG 240
V K P P P G K S P A K F T P I E K M G V
230 240

AGGACTGCTGAGCAGGCTGCGGCTACTTTGGGGATCCTGCTGTTCGTTGAGGTGCCGGGT 300
R T A E Q A A A T L G I L L F V E V P G
250 260

GGGTTGACGGTGGCCAAGGCGCTGGAGCTGTTTCATGCGAGTGGTGGAAATAGGTAGTT 360
G L T V A K A L E L F H A S G G K *
270 280

TTGCAGGTATACCTGCATGGGTAAATGTAAAAGTCGAATAAAAATGTCACAGAGTGACGG 420

ACTGATATAAATAAATTAATAAACATGTCATCATGAGTGACAGACTGATATAAATAAATA20 499

17. Method according to claim 15 or 16, wherein the pathogen is a fungus.

18. Method according to claim 17, wherein the fungus is selected from: Trichoderma reesei, Rhizoctonia solani or Fusarium sporotrichioides.

19. A plant cell capable of inhibiting the growth of a fungus pathogen being transformed such that it comprises in its genome the DNA according to claim 4 or a similar DNA sequence derived therefrom by nucleotide substitution, and which codes for the protein according to claim 7, wherein naturally occurring plant cells are excluded.

20. Plant cell according to claim 19, wherein the plant cell has been transformed with a DNA comprising the open reading frame of the following DNA sequence which has been completed in the 5'-region by a corresponding cDNA clone:

GCGGTGACGACGCTGCTCCTCATGGTGAACGAGGCCACGCGGTTCCAGACGGTGTCGGGG 60
A V T T L L L M V N E A T R F Q T V S G
170 180

TTCGTGGCCGGGCTGCTGCACCCCAAGGCGGTGGAGAAGAAGAGCGGGAAGATCGGCAAT 120
F V A G L L H P K A V E K K S G K I G N
190 200

GAGATGAAGGCCCAGGTGAACGGGTGGCAGGACCTGTCCGCGGCGCTGCTGAAGACGGAC 180
E M K A Q V N G W Q D L S A A L L K T D
210 220

GTGAAGCCCCCGCCGGGAAAGTCGCCAGCGAAGTTCACGCCGATCGAGAAGATGGGCGTG 240
V K P P P G K S P A K F T P I E K M G V
230 240

AGGACTGCTGAGCAGGCTGCGGCTACTTTGGGGATCCTGCTGTTCGTTGAGGTGCCGGGT 300
R T A E Q A A A T L G I L L F V E V P G
250 260

GGGTTGACGGTGGCCAAGGCGCTGGAGCTGTTTCATGCGAGTGGTGGAAAATAGGTAGTT 360
G L T V A K A L E L F H A S G G K *
270 280

TTGCAGGTATACCTGCATGGGTAAATGTAAAAGTCGAATAAAAATGTCACAGAGTGACGG 420

ACTGATATAAATAAATTAATAAACATGTCATCATGAGTGACAGACTGATATAAATAAATA20 499

21. Plant cell according to claim 19 or 20, which is a cell of a monocotyl plant, a cereal plant, a maize plant, a dicotyl plant, a Solanacee or Cucurbitacee.

22. Plants or parts of plants, consisting essentially of plant cells according to one according to claim 19 or 20.

23. Plant according to claim 22, which is selected from a monocotyl plant, a cereal plant, a maize plant, a dicotyl plant, a Solanacee or a Cucurbitacee.

## Revendications

1. ADN qui code une protéine qui comprend la séquence d'acides aminés suivante :

                                                M  A  A  K  M  A
                                               -1  1

K  N  V  D  K  P  L  F  T  A  T  F  N  V  Q  A  S  S  A  D
            10                            20

Y  A  T  F  I  A  G  I  R  N  K  L  R  N  P  A  H  F  S  H
            30                            40

N  R  P  V  L  P  P  V  E  P  N  V  P  P  S  R  W  F  H  V
            50                            60

V  L  K  A  S  P  T  S  A  G  L  T  L  A  I  R  A  D  N  I
            70                            80

Y  L  E  G  F  K  S  S  D  G  T  W  W  E  L  T  P  G  L  I
            90                           100

P  G  A  T  Y  V  G  F  G  G  T  Y  R  D  L  L  G  D  T  D
           110                           120

K  L  T  N  V  A  L  G  R  Q  Q  L  A  D  A  V  T  A  L  H
           130                           140

G R T K A D K P S G P K Q Q Q A R E A V
150 160

T T L L M V N E A T R F Q T V S G F V
170 180

A G L L H P K A V E K K S G K I G N E M
190 200

K A Q V N G W Q D L S A A L L K T D V K
210 220

P P P G K S P A K F A P I E K M G V R T
230 240

A V Q A A N T L G I L L F V E V P G G L
250 260

T V A K A L E L F H A S G G K *

2. Séquence selon la revendication 1 comprenant la séquence nucléotidique de cadre de lecture ouvert :

CTTAATAGCACATCTTGTCCGTCTTAGCTTTGCATTACATCCATGGCGGCAAAGATGGCG 60
*                    M  A  A  K  M  A
-1 1

AAGAACGTGGACAAGCCGCTCTTCACCGCGACGTTCAACGTCCAGGCCAGCTCCGCCGAC 120
K  N  V  D  K  P  L  F  T  A  T  F  N  V  Q  A  S  S  A  D
10                            20

TACGCCACCTTCATCGCCGGCATCCGCAACAAGCTCCGCAACCCGGCGCACTTCTCCCAC 180
Y  A  T  F  I  A  G  I  R  N  K  L  R  N  P  A  H  F  S  H
30                            40

AACCGCCCCGTGCTGCCGCCGGTCGAGCCCAACGTCCCGCCGAGCAGGTGGTTCCACGTC 240
N  R  P  V  L  P  P  V  E  P  N  V  P  P  S  R  W  F  H  V
50                            60

GTGCTCAAGGCCTCGCCGACCAGCGCCGGGCTCACGCTGGCCATTCGGGCGGACAACATC 300
V  L  K  A  S  P  T  S  A  G  L  T  L  A  I  R  A  D  N  I
70                            80

TACCTGGAGGGCTTCAAGAGCAGCGACGGCACCTGGTGGGAGCTCACCCCGGGCCTCATC 360
Y  L  E  G  F  K  S  S  D  G  T  W  W  E  L  T  P  G  L  I
90                            100

CCCGGCGCCACCTACGTCGGGTTCGGCGGCACCTACCGCGACCTCCTCGGCGACACCGAC 420
P  G  A  T  Y  V  G  F  G  G  T  Y  R  D  L  L  G  D  T  D
110                           120

AAGCTGACCAACGTCGCTCTCGGCCGGCAGCAGCTGGCGGACGCGGTGACCGCCCTCCAC 480
K  L  T  N  V  A  L  G  R  Q  Q  L  A  D  A  V  T  A  L  H
130                           140

GGGCGCACCAAGGCCGACAAGCCGTCCGGCCCGAAGCAGCAGCAGGCGAGGGAGGCGGTG 540
G R T K A D K P S G P K Q Q Q A R E A V
150 160

ACGACGCTGCTCCTCATGGTGAACGAGGCCACGCGGTTCCAGACGGTGTCTGGGTTCGTG 600
T T L L M V N E A T R F Q T V S G F V
170 180

GCCGGGTTGCTGCACCCCAAGGCGGTGGAGAAGAAGAGCGGGAAGATCGGCAATGAGATG 660
A G L L H P K A V E K K S G K I G N E M
190 200

AAGGCCCAGGTGAACGGGTGGCAGGACCTGTCCGCGGCGCTGCTGAAGACGGACGTGAAG 720
K A Q V N G W Q D L S A A L L K T D V K
210 220

CCTCCGCCGGGAAAGTCGCCAGCGAAGTTCGCGCCGATCGAGAAGATGGGCGTGAGGACG 780
P P P G K S P A K F A P I E K M G V R T
230 240

GCTGTACAGGCCGCCAACACGCTGGGGATCCTGCTGTTCGTGGAGGTGCCGGGTGGGTTG 840
A V Q A A N T L G I L L F V E V P G G L
250 260

[?] GGTGGCCAAGGCGCTGGAGCTGTTCCATGCGAGTGGTGGGAAATAGGTAGTTTTCCAG 900
T V A K A L E L F H A S G G K *

[?]TATACCTGCATGGGTAGTGTAAAAGTCGAATAAACATGTCACAGAGTGACGGACTGATA 960

[?]AAATAAATAAATAAACGTGTCACAGAGTTACATATAAACAAATAAATAAATAATTAAAA 1020

TGTCCAGTTTA47 1078

3. ADN selon la revendication 1, comprenant la séquence nucléotidique suivante :

CTTAATAGCACATCTTGTCCGTCTTAGCTTTGCATTACATCCATGGCGGCAAAGATGGCG 60

AAGAACGTGGACAAGCCGCTCTTCACCGCGACGTTCAACGTCCAGGCCAGCTCCGCCGAC 120

TACGCCACCTTCATCGCCGGCATCCGCAACAAGCTCCGCAACCCGGCGCACTTCTCCCAC 180

AACCGCCCCGTGCTGCCGCCGGTCGAGCCCAACGTCCCGCCGAGCAGGTGGTTCCACGTC 240

GTGCTCAAGGCCTCGCCGACCAGCGCCGGGCTCACGCTGGCCATTCGGGCGGACAACATC 300

TACCTGGAGGGCTTCAAGAGCAGCGACGGCACCTGGTGGGAGCTCACCCCGGGCCTCATC 360

CCCGGCGCCACCTACGTCGGGTTCGGCGGCACCTACCGCGACCTCCTCGGCGACACCGAC 420

AAGCTGACCAACGTCGCTCTCGGCCGGCAGCAGCTGGCGGACGCGGTGACCGCCCTCCAC 480

GGGCGCACCAAGGCCGACAAGCCGTCCGGCCCGAAGCAGCAGCAGGCGAGGGAGGCGGTG 540

ACGACGCTGCTCCTCATGGTGAACGAGGCCACGCGGTTCCAGACGGTGTCTGGGTTCGTG 600

GCCGGGTTGCTGCACCCCAAGGCGGTGGAGAAGAAGAGCGGAAGATCGGCAATGAGATG 660

AAGGCCCAGGTGAACGGGTGGCAGGACCTGTCCGCGGCGCTGCTGAAGACGGACGTGAAG 720

CCTCCGCCGGGAAAGTCGCCAGCGAAGTTCGCGCCGATCGAGAAGATGGGCGTGAGGACG 780

GCTGTACAGGCCGCCAACACGCTGGGGATCCTGCTGTTCGTGGAGGTGCCGGGTGGGTTG 840

ACGGTGGCCAAGGCGCTGGAGCTGTTCCATGCGAGTGGTGGGAAATAGGTAGTTTTCCAG 900

GTATACCTGCATGGGTAGTGTAAAAGTCGAATAAACATGTCACAGAGTGACGGACTGATA 960

TAAATAAATAAATAAACGTGTCACAGAGTTACATATAAACAAATAAATAAATAATTAAAA 1020

ATGTCCAGTTTA47 1078

4. ADN selon la revendication 1 ou 2, qui est fusionné avec un promoteur actif dans les plantes.

5. ADN selon la revendication 4 où le promoteur est choisi parmi : les promoteurs inductibles par des agents pathogènes, les promoteurs constitutifs, les promoteurs spécifiques du développement, les promoteurs spécifiques d'organes, les promoteurs inductibles et le promoteur wun1.

6. Vecteur d'expression d'ADN comprenant l'ADN selon l'une des revendications 1 à 5.

7. Protéine comprenant la séquence d'acides aminés :

M A A K M A
-1 1

K N V D K P L F T A T F N V Q A S S A D
10 20

Y A T F I A G I R N K L R N P A H F S H
30 40

N R P V L P P V E P N V P P S R W F H V
50 60

V L K A S P T S A G L T L A I R A D N I
70 80

Y L E G F K S S D G T W W E L T P G L I
90 100

P G A T Y V G F G G T Y R D L L G D T D
110 120

K L T N V A L G R Q Q L A D A V T A L H
130 140

G R T K A D K P S G P K Q Q Q A R E A V
150 160

T T L L L M V N E A T R F Q T V S G F V
170 180

A G L L H P K A V E K K S G K I G N E M
190 200

K A Q V N G W Q D L S A A L L K T D V K
210 220

[?] P P G K S P A K F A P I E K M G V R T
230 240

A V Q A A N T L G I L L F V E V P G G L
250 260

T V A K A L E L F H A S G G K

8. Utilisation de la protéine selon la revendication 7 pour inhiber la croissance d'agents pathogènes pour les plantes.

9. Utilisation de la protéine selon la revendication 7 pour obtenir des propriétés d'inhibition des agents pathogènes dans une plante.

10. Utilisation selon la revendication 8 ou 9 où l'agent pathogène est un champignon.

11. Utilisation selon la revendication 10 où le champignon est choisi parmi : Trichoderma reesei, Rhizoctonia solani et Fusarium sporotrichioides.

12. Procédé pour inhiber la croissance d'un champignon pathogène pour des plantes, comprenant l'application d'une protéine selon la revendication 7 sur le champignon.

13. Procédé selon la revendication 12 où une glucanase BGL et/ou une chitinase CHI de grains d'orge sont appliquées aussi sur le champignon.

14. Procédé selon la revendication 12 ou 13 où l'application sur le champignon a lieu par expression dans une plante transgénique.

15. Procédé pour produire des plantes résistantes aux agents pathogènes comprenant la transformation d'une plante avec l'ADN selon la revendication 4 ou une séquence d'ADN analogue, dérivée de celui-ci par substitution de nucléotides, qui code la protéine selon la revendication 7.

16. Procédé selon la revendication 15 où la plante a été transformée avec le cadre de lecture ouvert de la séquence d'ADN suivante, qui a été complétée dans le domaine 5' avec un clone d'ADNc correspondant :

GCGGTGACGACGCTGCTCCTCATGGTGAACGAGGCCACGCGGTTCCAGACGGTGTCGGGG 60
A V T T L L L M V N E A T R F Q T V S G
170 180

TTCGTGGCCGGGCTGCTGCACCCCAAGGCGGTGGAGAAGAAGAGCGGGAAGATCGGCAAT 120
F V A G L L H P K A V E K K S G K I G N
190 200

GAGATGAAGGCCCAGGTGAACGGGTGGCAGGACCTGTCCGCGGCGCTGCTGAAGACGGAC 180
E M K A Q V N G W Q D L S A A L L K T D
210 220

GTGAAGCCCCCGCCGGGAAAGTCGCCAGCGAAGTTCACGCCGATCGAGAAGATGGGCGTG 240
V K P P P G K S P A K F T P I E K M G V
230 240

AGGACTGCTGAGCAGGCTGCGGCTACTTTGGGGATCCTGCTGTTCGTTGAGGTGCCGGGT 300
R T A E Q A A A T L G I L L F V E V P G
250 260

GGGTTGACGGTGGCCAAGGCGCTGGAGCTGTTTCATGCGAGTGGTGGAAAATAGGTAGTT 360
G L T V A K A L E L F H A S G G K *
270 280

TTGCAGGTATACCTGCATGGGTAAATGTAAAAGTCGAATAAAAATGTCACAGAGTGACGG 420

CTGATATAAATAAATTAATAAACATGTCATCATGAGTGACAGACTGATATAAATAAATA20 499

17. Procédé selon la revendication 15 ou 16 où l'agent pathogène est un champignon.

18. Procédé selon la revendication 17 où le champignon est choisi parmi : Trichoderma reesei, Rhizoctonia solani et Fusarium sporotrichioides.

19. Cellule végétale qui peut inhiber la croissance d'un champignon pathogène, qui a été transformée de telle manière qu'elle contient dans son génome l'ADN selon la revendication 4 ou une séquence d'ADN analogue, dérivée de celui-ci par substitution de nucléotides et qui code la protéine selon la revendication 7; où les cellules végétales naturelles sont exclues.

20. Cellule végétale selon la revendication 19 où la cellule végétale a été transformée avec un ADN comprenant le cadre de lecture ouvert de la séquence d'ADN suivante qui a été complétée dans le domaine 5' par un clone d'ADNc correspondant :

GCGGTGACGACGCTGCTCCTCATGGTGAACGAGGCCACGCGGTTCCAGACGGTGTCGGGG 60
A V T T L L L M V N E A T R F Q T V S G
170 180

TTCGTGGCCGGGCTGCTGCACCCCAAGGCGGTGGAGAAGAAGAGCGGGAAGATCGGCAAT 120
F V A G L L H P K A V E K K S G K I G N
190 200

GAGATGAAGGCCCAGGTGAACGGGTGGCAGGACCTGTCCGCGGCGCTGCTGAAGACGGAC 180
E M K A Q V N G W Q D L S A A L L K T D
210 220

GTGAAGCCCCCGCCGGGAAAGTCGCCAGCGAAGTTCACGCCGATCGAGAAGATGGGCGTG 240
V K P P P G K S P A K F T P I E K M G V
230 240

AGGACTGCTGAGCAGGCTGCGGCTACTTTGGGGATCCTGCTGTTCGTTGAGGTGCCGGGT 300
R T A E Q A A A T L G I L L F V E V P G
250 260

GGGTTGACGGTGGCCAAGGCGCTGGAGCTGTTTCATGCGAGTGGTGGGAAATAGGTAGTT 360
G L T V A K A L E L F H A S G G K *
270 280

[?][?]GCAGGTATACCTGCATGGGTAAATGTAAAAGTCGAATAAAAATGTCACAGAGTGACGG 420

CTGATATAAATAAATTAATAAACATGTCATCATGAGTGACAGACTGATATAAATAAATA20 499

21. Cellule végétale selon la revendication 19 ou 20 qui est une cellule d'une plante monocotylédone, d'une plante de type céréale, d'une plante de type maïs, d'une plante dicotylédone, d'une solanacée ou d'une cucurbitacée.

22. Plantes ou parties de plantes qui consistent essentiellement en les cellules végétales selon l'une des revendications 19 et 20.

23. Plante selon la revendication 22 qui est choisie parmi une plante monocotylédone, une plante de type céréale, une plante de type maïs, une plante dicotylédone, une solanacée ou une cucurbitacée.
